# EUROPEAN PATENT APPLICATION

(11) **EP 3 858 478 A1**
(43) Date of publication of application: **04.08.2021**
(21) Application number: 20154776.7
(22) Date of filing: 31.01.2020
(51) Int. Cl.: B01J 21/08, B01J 21/10, B01J 23/14, B01J 23/30, B01J 29/035, B01J 29/16, B01J 35/00, B01J 37/00, B01J 37/02, B01J 37/03

(54) **STABLE, HIGH SELECTIVITY CATALYSTS AND CATALYST SYSTEMS, AND PROCESSES FOR THEIR USE**

(71) Applicant: SCG Chemicals Co., Ltd., Bangkok 10800 (TH)
(72) Inventor: Suriye, Kongkiat, 10270 Samut-Prakan (TH); Wannakao, Sippakorn, 40000 Khonkaen (TH); Jareewatchara, Wuttithep, 10310 Bangkok (TH)
(74) Representative: Erbacher, Martin

(57) **Abstract**

The present invention relates to catalysts, catalyst systems, and processes for the production of valuable light olefins, such as C₂-C₄ olefins (ethylene, propylene, and/or butenes) from paraffinic hydrocarbons, such as propane, through dehydrogenation and metathesis. Some particular aspects relate to the discovery of non-precious metal catalysts and catalyst systems utilizing such catalysts, for example in the case of being in an admixture with a metathesis catalyst, which advantageously exhibit high performance in terms of activity, selectivity, and stability. Other advantages can include a reduced production of byproducts (*e.g*., methane and ethane) that result from undesired side reactions, in addition to benefits that may be attained through the addition of a sulfur-bearing compound (*e.g.*, H₂S).

## Description

### FIELD OF THE INVENTION

The invention relates to catalyst systems comprising a dehydrogenation catalyst and an olefin metathesis catalyst, in addition to dehydrogenation and olefin metathesis processes for converting a paraffinic hydrocarbon (*e.g.,* propane) to at least two olefinic hydrocarbons (*e.g.,* ethylene and butene) having different carbon numbers, relative to the paraffinic hydrocarbon.

### DESCRIPTION OF RELATED ART

Olefins are important building blocks having value in many chemical industries, particularly in the production of plastics (*e.g.,* polyolefins), as well as specialty chemicals such as solvents, detergents, and adhesives. In the case of ethylene, for example, the global demand for this light olefin is currently high and expected to increase, in view of anticipated growth in the major end product polyethylene, used in plastic bags, films, bottles, *etc.* across a wide spectrum of applications. Intermediate products made from ethylene include ethylene oxide and ethylene glycol for the production of polyethylene terephthalate (PET) resins for PET fiber, as well as ethylene dichloride for the production of polyvinylchloride (PVC) plastic used in construction and piping. The increasing uses of these and other ethylene-based intermediates are expected to further increase the demand for ethylene. For example, the production of textile fibers from ethylene oxide is growing significantly, especially in the Asia-Pacific region. Also, producers of ethylene oxide have been able to profit from the worldwide growing substitution of glass by PET bottles and containers. Overall, the global ethylene market is regarded as being segmented into high density polyethylene (HDPE), low-density polyethylene (LDPE) and linear low density polyethylene (LLDPE) end products, as well as the above-noted intermediates, mainly used for the production of plastics.

A significant route for the production of low carbon number olefins is through steam cracking of higher carbon number paraffinic hydrocarbons, the main sources of which are obtained from crude oil refining. These include light gas oil, liquefied petroleum gas (LPG), naphtha, and other hydrocarbon fractions. A catalytic naphtha cracking process of commercial importance, using known high severity conditions to achieve the targeted production of light olefins, is described in US 6,867,341. Steam cracking, however, involves a very complex combination of reaction and gas recovery systems. Feedstock is charged to a thermal cracking zone in the presence of steam at effective conditions to produce a pyrolysis reactor effluent gas mixture. The mixture is then stabilized and separated into purified components through a sequence of cryogenic and conventional fractionation steps. Generally, the product ethylene is recovered as a low boiling fraction, such as an overhead stream, from an ethylene/ethane splitter column requiring a large number of theoretical stages due to the similar relative volatilities of the ethylene and ethane being separated. The production of olefins in steam cracking units, from hydrocarbon feedstocks such as C₄ mixtures obtained in refineries, is described in US 6,858,133; US 7,087,155; and US 7,375,257. Other significant sources of ethylene include byproducts of fluid catalytic cracking (FCC) and resid fluid catalytic cracking (RFCC), normally targeting gasoline production. FCC is described, for example, in US 4,288,688 and elsewhere. In recent years, a surge in shale gas production has led to ethane being a major potential source, or feedstock, of ethylene through dehydrogenation.

Yields of ethylene and other light olefins from steam cracking and other processes may be improved using known methods for the metathesis or disproportionation of C₄ and heavier olefins. Metathesis may also be combined with a separate cracking step in the presence of a zeolitic catalyst, as described, for example, in US 5,026,935 and US 5,026,936. Light olefins, namely propylene and butene that can be used for ethylene production through metathesis and/or cracking, can also be produced through a dedicated process of paraffin dehydrogenation, as described in US 3,978,150 and elsewhere. However, the significant capital cost of a propane dehydrogenation plant is normally justified only in cases of large-scale propylene production units (*e.g.,* typically 250,000 metric tons per year or more). The substantial supply of propane feedstock required to maintain this capacity is typically available from propane-rich liquefied petroleum gas (LPG) streams from gas plant sources.

The production of light olefins directly from paraffins through a combination of dehydrogenation and metathesis (disproportionation) reaction steps is described in US 3,445,541. For the initial dehydrogenation step, this reference discloses supported catalysts in which platinum or chromium oxide is used for dehydrogenation activity. Other dehydrogenation catalysts, referred to in this disclosure, are all based on chromium oxide. Also, in processes known for dedicated or "on-purpose" dehydrogenation of propane to target the production of propylene, considered the second most important olefin in the refining and chemical industries, the catalysts in commercial use rely on platinum (Pt) and/or chromium (Cr), generally in the form of chromium oxide (Cr₂O₃). The desire for light olefins from alternative, non-petroleum based feeds has also led to the use of oxygenates such as alcohols and, more particularly, methanol, ethanol, and higher alcohols or their derivatives. Methanol, in particular, is useful in a methanol-to-olefin (MTO) conversion process described, for example, in US 5,914,433. The yield of light olefins from such processes may be improved using olefin cracking to convert some or all of the C₄⁺ product of an MTO process in an olefin cracking reactor, as described in US 7,268,265. An oxygenate to light olefins conversion process in which the yields of olefin products of varying carbon numbers may be adjusted through the use of dimerization and metathesis is described in US 7,586,018.

Despite the use of various dedicated and non-dedicated routes for generating ethylene and other light olefins industrially, there remains an ongoing need for olefin production processes that can be carried out in a straightforward manner, with reduced complexity that leads to improved economics. In this regard, paraffin dehydrogenation is a favored reaction pathway, but the competitiveness of this production route depends highly on the catalyst used and its product yield and stability profile, in addition to its cost and safety characteristics. Whereas supported Pt and supported Cr₂O₃ catalysts have been in commercial use for a significant length of time, platinum and chromium pose considerable drawbacks in terms of, respectively, their cost and potential environmental impact. That is, conventional dehydrogenation catalysts are associated with significant limitations because Pt is expensive while Cr is toxic, such that neither of these dehydrogenation active metals is completely satisfactory. Moreover, such conventional catalyst types suffer from poor stability, or rapid deactivation due to coke (carbon) deposition and blockage of active metal sites, and/or metal sintering. To compensate for activity loss, reaction temperature can be increased, but with the associated drawback of a reduction in selectivity. Alternatively, continuous catalyst regeneration and replacement of spent catalyst with fresh (regenerated) catalyst has been employed to address deactivation, but with the additional requirement for equipment to transport solid catalyst from reactor to regenerator, as well as increased catalyst attrition rates resulting from this movement.

More recently, in J. CATAL. 344 (2016): 606-608 and J. CATAL. 351 (2017): 90-94, the art has proposed catalysts with tin (Sn) embedded into mesoporous silica, for propane dehydrogenation to propylene. However, a process directed to the production of all or predominantly all of this olefin would be susceptible to changing market forces that impact its overall supply, and consequently its price. For example, a projected rise in propane dehydrogenation capacity in China (CHEM. REV. 2014) could significantly reduce the value of propylene as a product, throughout the Asia-Pacific region. A similar scenario could result from the increasing trend toward ethane cracking and propane dehydrogenation, due to technological advances in shale gas processing, which would simultaneously cause a shortage of other specific products of naphtha cracking in the near future.

Improvements in processes and associated catalysts for the production of light olefins, particularly ethylene, propylene, and butenes, which can simultaneously address the above-noted challenges relating to cost, safety, performance, and flexibility, are continually being sought.

It is, therefore, the object of the present invention to provide a paraffin dehydrogenation and metathesis catalyst system and a paraffin dehydrogenation in metathesis process overcoming drawbacks of the prior art, in particular addressing the above-noted challenges relating to cost, safety, performance, and flexibility.

### SUMMARY OF THE INVENTION

The above object is achieved by a paraffin dehydrogenation and metathesis catalyst system comprising: a dehydrogenation catalyst comprising a stannosilicate, and a metathesis catalyst comprising one or more olefin metathesis active metals selected from the group consisting of metals in Group 6 and Group 7 of the periodic table, wherein said one or more olefin metathesis active metals are supported on an olefin metathesis solid support.

The stannosilicate may comprise tin being incorporated into a silicate or aluminosilicate framework.

The tin may be present in the dehydrogenation catalyst in an amount from about 3 wt-% to about 15 wt-%.

The dehydrogenation catalyst may further comprise one or more additional metals selected from the group consisting of In, Pb, Ge, Ga, Tl, Cu, Ag, Au, and mixtures thereof, being present in an amount, or combined amount, from about 0.1 wt-% to about 6 wt-%.

The one or more olefin metathesis active metals may be selected from the group consisting of W, Mo, and Re.

The dehydrogenation catalyst and the metathesis catalyst may be physically mixed in a catalyst bed.

The paraffin dehydrogenation and metathesis catalyst system may further comprise a second catalyst bed comprising substantially all of the metathesis catalyst.

The dehydrogenation catalyst and the metathesis catalyst may be in separate catalyst beds.

The paraffin dehydrogenation and metathesis catalyst system may comprise three or more of said separate catalyst beds, alternatingly may comprise said dehydrogenation catalyst and the metathesis catalyst.

The paraffin dehydrogenation and metathesis catalyst system may be a paraffin dehydrogenation and metathesis catalyst comprising: one or more dehydrogenation active metals selected from the group consisting of metals in Group 11, Group 13, and Group 14 of the periodic table, wherein said one or more dehydrogenation active metals are present in a dehydrogenation and metathesis solid support, and one or more olefin metathesis active metals supported on said dehydrogenation and metathesis catalyst support and selected from the group consisting of metals in Group 6 and Group 7 of the periodic table.

Said one or more olefin metathesis active metals may be present in the catalyst in an amount, or combined amount, from about 0.05 wt-% to about 15 wt-%.

At least a portion of said one or more dehydrogenation active metals may be dispersed on said dehydrogenation and metathesis solid support.

At least a portion of said one or more dehydrogenation active metals may be chemically incorporated into said dehydrogenation and metathesis solid support.

Said one or more dehydrogenation active metals may include tin and said dehydrogenation and the metathesis catalyst support may comprise a stannosilicate, wherein at least a portion of said tin may be incorporated into a silicate framework of said stannosilicate.

The tin may be present in the catalyst in an amount from about 0.02 wt-% to about 30 wt-%.

The paraffin dehydrogenation and metathesis catalyst system may be a paraffin dehydrogenation and metathesis catalyst system comprising at least one catalyst bed comprising a physical mixture of: a dehydrogenation catalyst comprising one or more dehydrogenation active metals selected from the group consisting of metals in Group 11, Group 13, and Group 14 of the periodic table, wherein said one or more dehydrogenation active metals are supported on a dehydrogenation solid support, and a metathesis catalyst comprising one or more olefin metathesis active metals selected from the group consisting of metals in Group 6 and Group 7 of the periodic table, wherein said one or more olefin metathesis active metals are supported on an olefin metathesis solid support.

A combined amount of metals in Groups 8-10 of the periodic table may be less than about 0.1 wt-% in the dehydrogenation catalyst.

The dehydrogenation solid support may comprise a stannosilicate, having tin incorporated into a silicate framework.

The dehydrogenation solid support may comprise a dehydrogenation support metal oxide, a dehydrogenation support zeolite, or a combination thereof.

The dehydrogenation support metal oxide may be selected from the group consisting of alumina, silica, zirconia, titania, magnesia, calcium oxide, and mixtures thereof.

The dehydrogenation support metal oxide may be an oxide of a first metal selected from the group consisting of Li, Mg, Zn, Fe, Ca, Ni, Co, Mn, and Cu, and the dehydrogenation solid support may comprise a further dehydrogenation support metal oxide of a second metal selected from the group consisting of Al, Ga, Y, In, Fe, Co, Ni, Mn, Cr, Ti, V, Zr, and La.

The object is further achieved by a dehydrogenation and metathesis process, the process comprising contacting a feed comprising a paraffinic hydrocarbon with at least one catalyst bed comprising a physical mixture of: a dehydrogenation catalyst comprising one or more dehydrogenation active metals selected from the group consisting of metals in Group 11, Group 13, and Group 14 of the periodic table, wherein said one or more dehydrogenation active metals are supported on a dehydrogenation solid support, anda metathesis catalyst comprising one or more olefin metathesis active metals selected from the group consisting of metals in Group 6 and Group 7 of the periodic table, wherein said one or more olefin metathesis active metals are supported on an olefin metathesis solid support.

A combined amount of metals in Groups 8-10 of the periodic table may be less than about 0.1 wt-% in the dehydrogenation catalyst.

The dehydrogenation solid support may comprise a stannosilicate, having tin incorporated into a silicate framework.

The dehydrogenation solid support may comprise a dehydrogenation support metal oxide, a dehydrogenation support zeolite, or a combination thereof.

The dehydrogenation support metal oxide may be selected from the group consisting of alumina, silica, zirconia, titania, magnesia, calcium oxide, and mixtures thereof.

The dehydrogenation support metal oxide may be an oxide of a first metal selected from the group consisting of Li, Mg, Zn, Fe, Ca, Ni, Co, Mn, and Cu, and the dehydrogenation solid support may comprise a further dehydrogenation support metal oxide of a second metal selected from the group consisting of Al, Ga, Y, In, Fe, Co, Ni, Mn, Cr, Ti, V, Zr, and La.

The dehydrogenation and metathesis process may be a dehydrogenation and metathesis process comprising contacting a feed comprising a non-cyclic paraffinic hydrocarbon with a dehydrogenation and metathesis catalyst system comprising at least: a dehydrogenation catalyst comprising a stannosilicate.

At least a portion of the tin may be incorporated into a silicate or aluminosilicate framework.

The dehydrogenation and metathesis process may be a dehydrogenation and metathesis process, the process comprising contacting a feed comprising a paraffinic hydrocarbon, in the absence of an oxidizing agent with a dehydrogenation and metathesis catalyst system comprising at least: a dehydrogenation catalyst comprising a stannosilicate.

At least a portion of the tin may be incorporated into a silicate or aluminosilicate framework.

Said paraffinic hydrocarbon may be a C₂-C₁₂ paraffinic hydrocarbon.

Said paraffinic hydrocarbon may be propane.

The dehydrogenation and metathesis process may comprise contacting a feed comprising a paraffinic hydrocarbon with the dehydrogenation and metathesis catalyst system comprising at least one catalyst bed comprising a physical mixture of: a dehydrogenation catalyst comprising one or more dehydrogenation active metals selected from the group consisting of metals in Group 11, Group 13, and Group 14 of the periodic table, wherein said one or more dehydrogenation active metals are supported on a dehydrogenation solid support, and a metathesis catalyst comprising one or more olefin metathesis active metals selected from the group consisting of metals in Group 6 and Group 7 of the periodic table, wherein said one or more olefin metathesis active metals are supported on an olefin metathesis solid support.

The feed may comprise LPG or naphtha.

The dehydrogenation and metathesis catalyst system may be a fixed bed catalyst system or a moving bed catalyst system.

The feed may further comprise a sulfur-bearing compound to provide a total sulfur concentration in the feed from about 5 vol-ppm to about 500 vol-ppm.

The sulfur-bearing compound may be H₂S.

The H₂S may be generated from an organic sulfide precursor.

The tin may be present in the dehydrogenation catalyst in an amount of at least about 3% by weight.

A combined amount of metals in Groups 8-10 of the periodic table may be less than about 0.01 wt-% in the dehydrogenation catalyst.

A combined amount of metals in Groups 1 and 2 of the periodic table is less than about 0.01 wt-% in the dehydrogenation catalyst.

The object is also achieved by any combination of two or more of the above embodiments.

The present invention relates to catalysts, catalyst systems, and processes for the production of valuable light olefins, such as C₂-C₄ olefins (ethylene, propylene, and/or butenes) from paraffinic hydrocarbons, such as propane, through dehydrogenation and metathesis. Advantageously, catalysts and catalyst systems described herein can perform both of these reaction steps. In the case of a feed comprising propane, for example, its dehydrogenation to propylene and hydrogen proceeds according to:

C₃H₈ → C₃H₆ + H₂ (reaction 1-dehydrogenation).

The resulting propylene can then undergo metathesis or conversion to olefin products of lower and higher carbon numbers, in this case ethylene and butene, respectively. More particularly, olefin metathesis results in redistribution of alkylidene radicals that would be generated upon cleavage of the carbon-carbon double bond of an acyclic olefin. The self-metathesis of the asymmetrical olefin propylene, therefore, results in rearrangement of the olefinic carbon atom substituents to produce both ethylene and butene according to the following reaction: 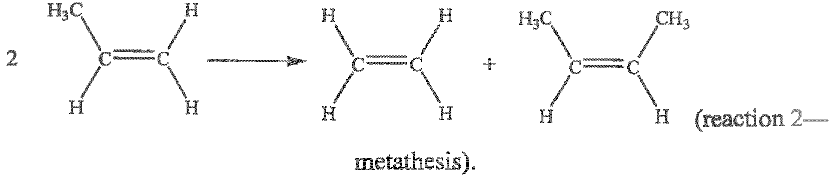 Other olefins can result, including other isomers of butene, besides cis-butene-2 (shown above), as well as higher olefins, such as C₅⁺ olefins.

Aspects of the invention relate to the discovery of non-precious metal catalysts and catalyst systems, for example catalysts and catalyst systems comprising no or substantially no (*e.g.,* less than about 0.1 wt-%) platinum. Alternatively, or in combination, such catalysts and catalyst systems may also comprise no or substantially no (*e.g.,* less than about 0.1 wt-%) chromium. Therefore, the expense and/or safety concerns associated with conventional catalysts for paraffin dehydrogenation can be avoided. Moreover, catalysts and catalyst systems described herein advantageously exhibit high performance in terms of activity, selectivity, and stability. For example, activity may be characterized by the ability of the catalyst to achieve a relatively high conversion of paraffinic hydrocarbons under even relatively mild operating conditions, such as relatively low temperatures and/or relatively high space velocity (corresponding to relatively low residence time). Selectivity may be characterized by the ability of the catalyst to convert paraffinic hydrocarbons (*e.g*., propane), at a given level of conversion, to products that include a relatively high percentage of desired olefinic hydrocarbons (*e.g.,* C₂-C₄ olefins), and a relatively limited percentage of undesired byproducts, such as byproduct hydrocarbons (*e.g.,* hydrocarbons resulting from cracking or hydrogenation, such as methane and ethane). A high selectivity can manifest in a high yield of desired olefinic hydrocarbons, as defined herein. Stability may be characterized by the ability of the catalyst to maintain constant performance over an extended operating period, with relatively small adjustments to the operating conditions (*e.g.,* temperature), or alternatively by the ability of the catalyst to exhibit relatively small rate of decline in performance (*e.g.,* conversion) over an extended operating period, with no adjustments to the operating conditions.

For example, in terms of yield and stability, aspects of the invention are associated with catalysts and catalyst systems described herein maintaining (i) a surprisingly low selectivity to, and yield of, methane and/or ethane, and/or (ii) a surprisingly high stability, such that conversion and selectivity values are substantially constant over an operating period, *e.g.,* period in which propane is converted to C₂-C₄ olefins, of at least 100 hours, at least 200 hours, at least 300 hours, or at least 500 hours. In view of the stability characteristics of such catalysts and catalyst systems, according to particular embodiments they may be used in fixed bed reactors, which may require little or no auxiliary equipment for transport of significant quantities of catalyst. Representative fixed bed reactors may include one or more catalyst beds as described herein, which may, under reaction conditions described herein, operate for example as isothermal fixed beds or adiabatic fixed beds. Multi-adiabatic fixed beds or multi-isothermal fixed beds are possible. Alternatively, moving bed systems may be employed, for example in conjunction with the continuous removal and regeneration of catalyst, *i.e.,* the catalysts and catalyst systems may be used in a continuous catalyst regeneration (CCR) reactor/regenerator configuration. As a further alternative, the catalyst or catalyst system may be in the form of a fluidized bed having favorable mass transfer and heat exchange characteristics.

Embodiments of the invention are directed to catalyst systems for paraffin dehydrogenation and metathesis, which such systems comprising both a dehydrogenation catalyst and a metathesis catalyst as described herein. These catalysts may be present in one or more beds that are disposed in one or more reactors, such as dehydrogenation reactors, dehydrogenation/metathesis reactors, or metathesis reactors, with the beds having various configurations, such as a physical mixture of the dehydrogenation catalyst and the metathesis catalyst, or possibly all or substantially all of the dehydrogenation catalyst and the metathesis catalyst (*e.g.,* in the case of alternating layers of the dehydrogenation catalyst and the metathesis catalyst). A particular embodiment of the invention is directed to a paraffin dehydrogenation and metathesis catalyst system comprising a physical mixture of (i) a dehydrogenation catalyst comprising one or more dehydrogenation active metals selected from the group consisting of metals in Groups 11, 13, and 14 (*e.g.,* a metal in either Group 13 or Group 14) of the periodic table, with such one or more dehydrogenation active metals being supported on a dehydrogenation solid support, and (ii) a metathesis catalyst comprising one or more olefin metathesis active metals selected from the group consisting of metals in Group 6 and Group 7 of the periodic table, with such one or more olefin metathesis active metals being supported on an olefin metathesis solid support.

Other embodiments of the invention are directed to dehydrogenation catalysts, as well as metathesis catalysts, as described herein. A particular embodiment of the invention is directed to a dehydrogenation catalyst comprising a stannosilicate (*e.g.,* a mesoporous stannosilicate), in which tin (Sn) is incorporated into a base silicate framework, such as a base zeolitic framework of a given structure type. Advantageously, such dehydrogenation catalysts can provide important benefits in terms of activity, selectivity, and/or stability as described herein. Without being bound by theory, it is believed that performance advantages over conventional catalysts, (*e.g.,* platinum-based catalysts having a promoter metal such as tin) result from the suppression or avoidance of detrimental alloying effects. In this regard, such dehydrogenation catalysts, in addition to catalyst systems comprising such dehydrogenation catalysts, may be characterized as having a substantial absence (*e.g.,* having less than about 0.1 wt-% of a combined amount) of metals such as chromium and/or metals in Groups 8-10 of the periodic table. According to such embodiments, these catalysts and catalyst systems may advantageously forego the use of elements of conventional catalysts that are relatively hazardous (*e.g.,* Cr) or expensive (*e.g.,* Pt).

Further embodiments of the invention are directed to dehydrogenation and metathesis catalysts, for example catalysts having a single type of support composition, or possibly a uniform composition. These catalysts may comprise (i) as a support, a stannosilicate (*e.g.,* a mesoporous stannosilicate), in which tin (Sn) is incorporated into a base silicate framework and (ii) one or more metals supported on the support. According to particular embodiments, tin may be present in the catalyst in an amount from about 3 wt-% to about 15 wt-%. Preferably, all or substantially all, such as at least about 95%, of the tin in the catalyst may be present in the stannosilicate support. These catalysts may further comprise one or more metals supported on the stannosilicate support, such as one or more metals selected from the group consisting of metals in Group 6 and Group 7 of the periodic table. These one or more metals may present in the catalyst in an amount, or combined amount, from about 1 wt-% to about 20 wt-%.

Yet further embodiments are directed to dehydrogenation and metathesis processes, with such processes comprising contacting a feed comprising a paraffinic hydrocarbon with any of (i) a dehydrogenation catalyst, (ii) a metathesis catalyst, (iii) a dehydrogenation and metathesis catalyst, or (iv) a catalyst system, as described herein. According to particular embodiments, the paraffinic hydrocarbon may be non-cyclic, such as in the case of an aliphatic paraffinic hydrocarbon or, more particularly, a straight-chain or branched paraffinic hydrocarbon. In more particular embodiments, the paraffinic hydrocarbon is a C₂-C₁₂ paraffinic hydrocarbon, such as a C₂-C₆ paraffinic hydrocarbon, with propane being representative. According to other particular embodiments, the paraffinic hydrocarbon is a straight-chain or branched C₄ paraffinic hydrocarbon, a straight-chain or branched C₅ paraffinic hydrocarbon, or a straight-chain or branched C₆ paraffinic hydrocarbon. The feed may comprise a mixture of any two or more paraffinic hydrocarbons, such as any two or more paraffinic hydrocarbons selected from C₂-C₆ paraffinic hydrocarbons, for example selected from C₂-C₅ paraffinic hydrocarbons or selected from C₂-C₄ paraffinic hydrocarbons. The feed may comprise, more particularly, all or substantially all propane, all or substantially all butane, or a mixture of all or substantially all propane and butane in combination, with butane being present as n-butane, i-butane, or a mixture of these isomers. A particular feed of interest is liquefied petroleum gas (LPG), which is an example of such mixture comprising substantially all propane and butane. The feed may comprise or consist of other hydrocarbon mixtures, which, like LPG, may be derived from crude oil refining or biofuel synthesis, and may generally have boiling points within a given temperature range, for example as a result of being recovered by fractionation (distillation). Representative mixtures are mixtures of C₂-C₁₂ hydrocarbons, C₂-C₈ hydrocarbons, C₃-C₆ hydrocarbons, C₄-C₁₂ hydrocarbons, C₄-C₈ hydrocarbons, C₄-C₆ hydrocarbons, C₅-C₁₂ hydrocarbons, C₅-C₈ hydrocarbons, and C₅-C₆ hydrocarbons. For example, the feed may comprise or consist of naphtha boiling range hydrocarbons (*i.e.*, a naphtha fraction), which may also be referred to as gasoline boiling range hydrocarbons. Such hydrocarbon mixtures may be characterized as having an initial boiling point (or "front-end") temperature characteristic of C₅ hydrocarbons, for example from about 30°C (86°F) to about 40°C (104°F), with a representative value being 35°C (95°F), and a distillation end point temperature range from about from about 110°C (230°F) to about 149°C (300°F), and typically from about 121°C (250°F) to about 143°C (290°F), with a representative value being 130°C (266°F). These boiling point temperatures may be measured according to ASTM D86, with the distillation end point temperature being the 95% recovery value. In other particular embodiments, in (i), (ii), (iii), or (iv) above, a combined amount of metals in Groups 8-10 of the periodic table is less than about 0.1 wt-%, such as less than about 0.01 wt-%. In yet other particular embodiments, in (i), (ii), (iii), or (iv) above, a combined amount of metals in Groups 1 and 2 of the periodic table is less than about 0.1 wt-%, such as less than about 0.01 wt-%. In some embodiments, the feed may be contacted with a platinum- and/or chromium-containing catalyst, or a catalyst bed comprising such catalyst, positioned upstream and/or downstream of (i), (ii), (iii), or (iv) above, such as catalyst system (iv). In the case of such upstream catalyst or catalyst bed, this may result in an upstream or initial conversion of the paraffinic hydrocarbon (*e.g.,* propane) to its corresponding olefin (*e.g.,* propylene), prior to contacting of the feed having this upstream or initial conversion with (i), (ii), (iii), or (iv) above. In the case of such downstream catalyst or catalyst bed, this may result in a downstream or final conversion of the paraffinic hydrocarbon (*e.g.,* propane) to its corresponding olefin (*e.g.,* propylene), after contacting of the feed with (i), (ii), (iii), or (iv) above, resulting in an upstream or initial conversion. In other embodiments, the feed may be contacted with any of (i), (ii), (iii), or (iv) above, in the absence of a platinum- and/or chromium-containing catalyst, or in the absence of a catalyst bed comprising such catalyst. For example, catalyst system (iv) may be used, in the absence of a platinum-containing catalyst, or in the absence of a catalyst bed comprising such catalyst.

Other aspects of the invention are associated with the surprising discovery that a feed further comprising one or more sulfur-bearing compounds, and preferably hydrogen sulfide (H₂S), can provide performance advantages. The advantages may be measured in comparison to the use of a reference feed that is the same in all respects but that lacks the sulfur-bearing compound, in a baseline process that is the same in all respects, including conditions (temperature, pressure, weight hourly space velocity). Such performance advantages may, in particular, include higher selectivity and yield of C₂-C₄ olefins and/or lower selectivity and yield of methane and/or ethane. Without being bound by theory, it is believed that such observed performance advantages can be attributed to the suppression of unwanted side reactions, such as the hydrogenation of ethylene to ethane, to increase the yield of the former and decrease the yield of the latter. According to other embodiments, advantages may reside in a decreased requirement for pretreating certain paraffinic hydrocarbon-containing feeds to remove sulfur. In particular, cost advantages may be realized in view of a reduced stringency, in terms of the maximum feed sulfur level due to sulfur tolerance of catalysts and catalyst systems described herein.

Various aspects of the invention therefore relate to important discoveries regarding feeds, as well as catalysts and catalyst systems, used in catalyzing dehydrogenation and metathesis reactions, described above. These reactions may proceed in combination (*e.g.,* sequentially) to produce, from a feed comprising one or more paraffinic hydrocarbons and optionally one or more sulfur-bearing compounds (*e.g.*, H₂S), one or more light olefins selected from the group consisting of C₂-C₆ monoolefins, for example selected from the group consisting of C₂-C₅ monoolefins. Representative paraffinic hydrocarbons include C₂-C₆ paraffins, such as ethane, propane, butane, pentane, and hexane, including all structural isomers of the latter three. C₂-C₆ monoolefins include ethylene, propylene, butene (including any of its positional and structural isomers, namely butene-1, cis-butene-2, trans-butene-2, and isobutylene), pentene (including any of its positional and structural isomers), and hexene (including any of its positional and structural isomers). Particular embodiments are directed to the production of ethylene, often in conjunction with one or more other olefins such as propylene, butene, and/or pentene and higher carbon number olefins (C₅⁺ olefins), from propane (*e.g.,* which may be present in the feed as a component of LPG). Representative processes therefore comprise contacting a feed comprising one or more paraffinic hydrocarbons such as propane with a dehydrogenation and metathesis catalyst or catalyst system as described herein.

According to particular embodiments, a paraffinic hydrocarbon (*e.g.,* propane) is present in the feed at a predominant concentration (*e.g.,* greater than about 50% by volume and/or greater than that of any paraffinic hydrocarbon having a different carbon number, such as butane), and the product resulting from the conversion of this paraffinic hydrocarbon comprises at least two olefinic hydrocarbons (*e.g.,* ethylene and butene) having different carbon numbers relative to the paraffinic hydrocarbon that is present in the feed at the predominant concentration. According to preferred embodiments, as a result of the metathesis, the selectivity to these olefinic hydrocarbons, or possibly even to only one of these olefinic hydrocarbons of particular interest/value (*e.g.,* ethylene), is at least about 5%, such as at least about 12% (*i.e.,* at least about 5%, such as at least about 12%, of the weight of the converted products is attributed to these olefinic hydrocarbons). Also, according to particular embodiments, this selectivity to at least one olefinic hydrocarbon may be achieved at a conversion of the paraffinic hydrocarbon, which is present in the feed at a predominant concentration as described above, of at least about 20%, such as at least about 40%.

Overall, aspects of the invention are associated with the discovery of feeds, catalysts, and catalyst systems that exhibit improved activity, selectivity, and/or stability in the production of olefinic hydrocarbons from paraffinic hydrocarbons, such as propane that may be a source of a relatively inexpensive feed. These and other advantages may be realized, relative to conventional dehydrogenation catalysts utilizing a precious metal (*e.g.,* platinum) or a hazardous metal (*e.g.,* chromium). With respect to improved selectivity, which results in improved yield of desired olefins (*e.g.,* C₂-C₄ olefins, and particularly ethylene and/or propylene), those skilled in the art will appreciate that even small increases in this respect can have a profound impact on the economic viability, attractiveness, and/or competitiveness of a hydrocarbon conversion process, especially considering a typical scale of commercial operation. The use of feeds, catalysts, and catalyst systems described herein can lead to significant yield increases of one or more desirable olefinic hydrocarbons such as ethylene, having a high demand and associated market value. With respect to improved stability, this can advantageously obviate the need for continuous or frequent catalyst regeneration, as well as the need for the associated equipment required to transport the catalyst from the reactor to a regenerator. Importantly, the combination of dehydrogenation and metathesis provides flexibility in terms of the ability to produce olefinic hydrocarbons of various carbon numbers, consistent with those obtained from naphtha cracking and having differing market values that can vary depending on a number of external factors.

These and other aspects and embodiments associated with the present invention are apparent from the following Detailed Description.

### BRIEF DESCRIPTION OF THE DRAWINGS

FIG. 1 depicts transmission electron microscopy (TEM) images of a mesoporous stannosilicate (tin-silica), prepared by a precipitation method described herein and having highly dispersed Sn within the silicate, as well as SnO₂ nanoparticles dispersed external to the mesopores.
FIG. 2 depicts transmission electron microscopy (TEM) images of a mesoporous stannosilicate (tin-silica), prepared by incipient wetness impregnation and having dispersed SnO₂ nanoparticles.
FIG. 3 is a graph that illustrates the propane conversion, selectivity to total olefins, and selectivity to paraffins, achieved over an extended operating period, in evaluating the performance of a catalyst system as described herein.
FIG. 4 is a graph that illustrates the selectivities over time to the component olefins and component paraffins, in the catalyst system evaluation that was used to obtain the operational results illustrated in FIG. 1.
FIG. 5 depicts hydrogen-temperature programmed reduction (H2-TPR) profiles for different types of catalysts having tin deposited on silica, with the same tin loading in each case.

### DETAILED DESCRIPTION

As discussed above, the present invention relates to embodiments including (i) dehydrogenation catalysts for performing dehydrogenation reactions such as reaction 1 above, (ii) metathesis catalysts for performing olefin metathesis reactions such as reaction 2 above, (iii) catalyst systems comprising dehydrogenation catalyst particles and olefin metathesis catalyst particles (*e.g.,* as a uniform mixture), in which at least a portion of one or both of these types of catalyst particles are particles of a dehydrogenation catalyst or an olefin metathesis catalyst as described herein (*e.g.,* according to embodiments (i) or (ii) above), and (iv) dehydrogenation and metathesis processes for performing a dehydrogenation reaction such as reaction 1 above in combination with (e.g., preceding) olefin metathesis reaction such as reaction 2 above, by contacting a feed comprising a paraffinic hydrocarbon with a catalyst system as described herein (*e.g.,* according to embodiment (iii) above). Such catalysts, catalyst systems, and processes are associated with unique advantages, particularly in terms of providing a high yield of olefinic hydrocarbons such as C₂-C₄ olefinic hydrocarbons in combination or such as ethylene in particular.

As used herein, generic terms for hydrocarbons having a particular carbon number are meant to encompass all double bond positional isomers and/or structural isomers (where applicable). For example, the terms "butene," "butenes," and "butene isomers" are meant to encompass butene-1, cis-butene-2, trans-butene-2, and isobutylene. The term "carbon number," in reference to olefinic or paraffinic hydrocarbons, for example in phrases such as "same carbon number" or "different carbon number," is meant to refer to the number of carbon atoms present in that olefinic or paraffinic hydrocarbon.

For element group designations described herein, reference is made to the "CRC Handbook of Chemistry and Physics", 76th Edition (1995-1996), by David R. Lide, published by CRC Press, Inc. (USA), in which the groups of the periodic table are numbered as Groups 1 to 18.

As used herein, the term "stannosilicate" refers to a base material comprising silica (SiO₂) or silicate anions (*e.g.,* SiO₄⁻⁴) and having tin deposited thereon, for example in the form of tin oxide (SnO₂) or elemental tin (Sn), or a combination of both forms, with the term "deposited thereon" in the case of a stannosilicate meaning that the tin is present within pores or channels of the base material and/or is present on the surface of the base material. In the case a base material comprising silicate anions, such base material may be in the form of a silicate or aluminosilicate framework, for example a zeolite framework having a zeolite structure type as described and referred to in Baerlocher Ch., McCusker L.B., and Olson, D.H., Atlas of Zeolite Framework Types, 6th Revised Ed., Elsevier (2007). For example, the base material may have, or may comprise a zeolite having, a SBA-15, BEA, MFI, FAU, FER, MWW, MOR, LTL, or MCM structure type. A specific base material may comprise, for example, silica having an SBA-15 structure type or an MCM-41 structure type. According to representative embodiments, with respect to a stannosilicate, (i) all or a portion of the tin may be chemically incorporated or bonded with the base material, particularly in the case of tin being present within pores or channels of the base material, and forming chemical bonds with the base material, optionally through "polymeric," or "oligomeric," -Sn-Sn- linkages, or -Sn-O-Sn- linkages. For example, in the case of a base material that is in the form of a silicate or aluminosilicate framework, a portion of the silicon (Si) atoms of tetrahedral silicates (SiO₄), or other tetrahedral lattice sites may be substituted by tin (Sn) atoms, which are therefore present in the stannosilicate as atomically or polymerically dispersed Sn, such as in the case of forming -Sn-O-Si- bonds or -O-Si-O(Sn-Sn-)ₙO-Si- bonds or -O-Si-O(Sn-O-Sn-)ₙO-Si- bonds. The value of "n" may be an integer such as from 1 to 100 or 1 to 20. According to other representative embodiments, with respect to a stannosilicate, (ii) all or a portion of the tin may be characterized according to its dispersion about the base material, particularly in the case of tin being present on the surface of the base material, for example in the form of Sn or SnO₂ particles. Such surface particles, for example SnO₂ surface particles, may comprise a quantity of tin in excess of an amount that is chemically bonded with the base material, according to (i) above. Therefore, a stannosilicate may be defined as having characteristic (i) above, *i.e*., according to which at least a portion of tin content of the stannosilicate is chemically bonded with the base material, or may be defined as having characteristic (ii) above, *i.e.,* according to which at least a portion of the tin content of the stannosilicate is well dispersed about (*e.g.,* on the surface of) the base material, or may be defined as having both characteristics (i) and (ii) above.

In some embodiments, both of the characteristics (i) and (ii) may be confirmed using hydrogen-temperature programmed reduction (H₂-TPR), which provides quantitative information of the reducibility of tin species of the stannosilicate. According to the H₂-TPR method, a reducing gas mixture of about 10 vol-% H₂ diluted in an inert gas such as N₂ or Ar is passed over a sample of the stannosilicate, at a predetermined rate of temperature increase and temperature range, typically 100°C to 900°C. Changes in the thermal conductivity of the gas stream are monitored using a thermal conductivity detector (TCD), and the signal from this instrument is converted to concentration of hydrogen, according to a known calibration. Integrating the area (or peak areas) under the concentration vs. temperature curve provides a measure of the total hydrogen consumed in reducing the tin species. In the reduction of tin in performing H₂-TPR, the hydrogen consumed can be divided into those amounts consumed over (a) the temperature range of 200°C to 450°C and corresponding to the reduction of Sn⁺⁴ to Sn⁺², and (b) the temperature range of 600°C to 800°C and corresponding to the reduction of Sn⁺⁴ or Sn⁺² to elemental or metallic tin, Sn⁰. In the case of a significant proportion of the tin being chemically bonded with the base material according to characteristic (i), such that a significant proportion of the tin is well dispersed about the base material according to characteristic (ii), the areas (or peak areas) within both temperature ranges (a) and (b) are relatively small, due to the chemical bonding of tin to form Sn-O-Si bonds with the base material (such that the tin is dispersed at the atomic level), thereby leaving only such relatively small amount of SnO₂ to be reduced. In representative embodiments, generally less than about 50%, typically less than about 30%, and often less than about 20%, of the tin content of a stannosilicate is reduced in performing H₂-TPR over a temperature range of 200°C to 800°C, or otherwise over the combined temperature ranges of 200°C to 450°C and 600°C to 800°C, or otherwise over the temperature range of 600°C to 800°C alone. In contrast, it has been determined that larger and less well dispersed tin particles, for example SnO₂ surface particles, are reduced in performing H₂-TPR, particularly the temperature range of 600°C to 800°C.

In some embodiments, characteristic (ii) may be confirmed by ascertaining particle sizes of Sn-containing particles, for example SnO₂ particles, on the surface of the base material or at least external to the pores of the base material. Particle sizes may be determined using transmission electron microscopy (TEM) techniques, including scanning TEM, high resolution TEM (HRTEM), high-angle annular dark-field scanning TEM (HAADF STEM), and energy dispersive X-ray spectroscopic (EDS) mapping. For example, the stannosilicate may comprise Sn and/or SnO₂ particles having an average particle size from about 1 to about 100 nanometers (nm), such as from about 5 to about 20 nm or from about 5 to about 10 nm. Such particle sizes may refer to particles dispersed on the surface of the base material (*e.g.,* SnO₂ surface particles), or otherwise may refer to particles dispersed on the surface and throughout the base material (*e.g.,* SnO₂ surface particles and SnO₂ sub-surface or internal particles).

Representative stannosilicates, for example having the characteristics (i) and/or (ii) above, may be further characterized as "mesoporous stannosilicates," according to which the base material has a pore size, or average pore size, in the range of 2 nanometers (nm) to 50 nm. Preferably, in the case of a mesoporous stannosilicate, at least about 80%, or at least about 90%, of its pore volume can be attributed to pores within this size range. In the case of a particular mesoporous stannosilicate, at least a portion (*e.g.,* at least about 10% or at least about 25%), a predominant amount (*e.g.,* at least about 50% or at least about 80%), or substantially all (*e.g.,* at least about 95%) of its Sn atoms are substituted into a framework of a base material having, or into a framework of a zeolite present in the base material and having, a mesoporous structure (*e.g.,* of any of the above structure types, including any of the above structure types that may be modified, such as by acid or base leaching, to obtain a modified structure type having a mesoporous structure), with an average pore size within this range. In the case of a mesoporous stannosilicate, or mesoporous tin silicate, tin may be incorporated into tetrahedral lattice sites of a mesoporous silica network or matrix (framework). Particular types of mesoporous silica include those having the SBA-15 or MCM-41 zeolite structure types (SBA-15 or MCM-41 mesoporous silica). Representative stannosilicates, for example having the characteristics (i) and/or (ii) above, may be further characterized as "microporous stannosilicates," according to which the base material has a pore size, or average pore size, of less than 2 nm. Preferably, in the case of a microporous stannosilicate, at least about 80%, or at least about 90%, of its pore volume can be attributed to pores within this size range. In the case of a particular microporous stannosilicate, at least a portion (*e.g.,* at least about 10% or at least about 25%), a predominant amount (*e.g.,* at least about 50% or at least about 80%), or substantially all (*e.g.,* at least about 95%) of its Sn atoms are substituted into a framework of a base material having, or into a framework of a zeolite present in the base material and having, a microporous structure (*e.g.,* of any of the above structure types, such as a microporous BEA structure type), with an average pore size within this range. Therefore, in the case of a microporous stannosilicate, or microporous tin silicate, tin may be incorporated into tetrahedral lattice sites of a microporous silica network or matrix (framework). Other representative stannosilicates, for example having the characteristics (i) and/or (ii) above, may be further characterized as "macroporous stannosilicates," according to which the base material has a pore size, or average pore size, of greater than 50 nm. Preferably, in the case of a macroporous stannosilicate, at least about 80%, or at least about 90%, of its pore volume can be attributed to pores of this size. The extent of substitution of its Sn atoms into a framework of the base material may be as described above with respect to a mesoporous stannosilicate or microporous stannosilicate. Yet other representative stannosilicates, for example having the characteristics (i) and/or (ii) above, may be further characterized as "non-porous stannosilicates," having an extent of substitution of its Sn atoms into a framework of the base material as described above with respect to a mesoporous stannosilicate or microporous stannosilicate. Representative stannosilicates having the characteristics (i) and/or (ii) above may therefore be selected from the group consisting of a mesoporous stannosilicate, a microporous stannosilicate, a macroporous stannosilicate, and a non-porous stannosilicate.

A "mixed metal oxide" as used, for example, in the phrases "dehydrogenation solid support mixed metal oxide" and "metathesis solid support mixed metal oxide" refers to a mixture of metal oxides, such as a mixture of magnesium oxide (MgO) and aluminum oxide (Al₂O₃). The term "mixed metal oxide" extends to layered double hydroxides, which are particular materials from which mixtures of metal oxides, corresponding to the hydroxides of the layered double hydroxide (LDH), can be obtained by thermal treatment. A mixed metal oxide obtained by thermal treatment (*e.g.,* calcination) of an LDH may also by referred to as a layered double oxide (LDO). A representative thermal treatment comprises heating the layered double hydroxide, in this case serving as a "precursor" of the mixed metal oxide, for an extended time (*e.g.,* from about 1 to about 15 hours, such as from about 1 to about 10 hours or from about 5 to about 10 hours) and at an elevated temperature (*e.g.,* from about 300°C to about 800°C, such as from about 500°C to about 750°C) to transform the precursor layered double hydroxide to a mixture of metal oxides (*i.e.,* the mixed metal oxide), which may or may not retain the layered structure of the precursor. Therefore, a "mixed metal oxide," according to this disclosure, may either be present as (*i.e.,* in the form of), or derived from, a layered double hydroxide (LDH). Layered double hydroxides and their preparation are described, for example, in WO 2016/120423. These are also known as anionic clays or hydrotalcite-like materials, having a unique structure with positively charged layers and charge-balancing anions and water interlayers. A general chemical formula of an LDH is:

[M^{y+}₁₋ₓM²⁺ₓ(OH)₂]^{a+}(A^{r-})ₙ · b(H₂O) (I).

In formula (I) above, M and M' are first and second metals that may be independently selected from alkali metals, alkaline earth metals, transition metals, and other metals. In representative embodiments, the first metal, M, may be selected from the group consisting of Li, Mg, Zn, Fe, Ca, Ni, Co, Mn, and Cu, with Ca and Mg being preferred. In other representative embodiments, the second metal, M', may be selected from the group consisting of Al, Ga, Y, In, Fe, Co, Ni, Mn, Cr, Ti, V, Zr, and La, with Al being preferred. In formula (I) above, A is an anion, examples of which include fluoride, chloride, bromide, iodide, carbonate, bicarbonate, hydrogen phosphate, dihydrogen phosphate, nitrate, nitrite, borate, sulfate, phosphate, and hydroxide, with carbonate and nitrate being preferred. In formula (I) above, the value of x is preferably from 0.1 to 0.9; the value of y, representing the charge of the first metal, M, is preferably 1 or 2; the value of z, representing the charge of the second metal, M', is preferably 3 or 4; the value of a is (1-x)·y + x·z - 2; the value of r, representing the charge of the anion, is preferably 1, 2, or 3 (giving charges of "r-" that are namely -1, -2, or -3, respectively); the value of n is a/r; and the value of b, representing the relative number of water molecules, is preferably from 0 to 10.

In some cases, ranges of ratios of two components (i) : (ii), ranges of ratios of three components (i) : (ii) : (iii), *etc.* are expressed using ranges of values for the individual components to which these ratios apply. For example, in the case of a range of weight ratios (i) : (ii) expressed as "1 : 1-3," the second component value may range from 1 to 3 parts by weight, relative to the first component, such that this range of weight ratios encompasses any weight ratio of (i) : (ii) from 1:1 to 1:3. Equivalently, in the case of weight ratios (i) : (ii) : (iii) expressed as "0.5-2 : 0-2 : 0.5-2," the first and third components may range from 0.5 to 2 parts by weight, relative to the second component, and relative to each other, and the second component may range from 0-2 parts by weight, relative to the first and third components. Therefore, in the particular case of the second component being 0, this range of weight ratios encompasses any weight ratio of (i):(iii) from 1:4 (or 0.5:2) to 4:1 (or 2:0.5).

Aspects of the invention relate to advantages, as described herein, associated with catalyst systems comprising both a dehydrogenation catalyst and a metathesis catalyst, in addition to advantages associated with non-precious metal dehydrogenation catalysts, for example lacking platinum, or lacking both platinum and chromium, in terms of cost, safety, and performance. Particular aspects relate to dehydrogenation catalysts comprising a stannosilicate, for example a mesoporous stannosilicate, having tin incorporated into a base framework (or simply a framework), such as base silicate framework (or simply a silicate framework). Particles of a dehydrogenation catalyst or metathesis catalyst, if spherical, may have a diameter suitable for use in a fixed bed or moving bed, for example, in the range generally from about 1 mm to about 10 mm, typically from about 1 mm to about 5 mm, and often from about 1 mm to about 3 mm. Catalyst particles of such catalysts having other geometries, and also suitable for use in a fixed bed or moving bed, include cylindrical catalyst particles (*e.g.,* when prepared by extrusion). If cylindrical, a dehydrogenation catalyst or metathesis catalyst may have a diameter within any of the ranges for diameter given above, with respect to spherical catalysts. For example, extrudates may be formed having diameters of 1.59 mm (1/16 inch), 3.18 mm (1/8 inch), or 6.35 mm (1/4 inch). Cylindrical catalysts may also have a length generally from about 1 mm to about 10 mm, typically from about 1 mm to about 5 mm, and often from about 1 mm to about 3 mm Alternatively, such catalysts may have particle sizes suitable for fluidized bed operation, for example in the range generally from about 10 µm to 1000 µm and typically from about 50 µm to about 500 µm.

### Dehydrosenation Catalysts

Representative dehydrogenation catalysts therefore comprise a dehydrogenation active catalytic component (*e.g.,* comprising one or more dehydrogenation active metals) that is supported on a dehydrogenation solid support, which serves as a carrier of, or base material for, this component. For example, the dehydrogenation active catalytic component may be dispersed uniformly or possibly non-uniformly (*e.g.,* preferentially near the outer surface) within the dehydrogenation solid support, and optional other support constituents. The dehydrogenation solid support may comprise a metal oxide such as aluminum oxide (alumina), silicon oxide (silica), zirconium oxide (zirconia), titanium oxide (titania), magnesium oxide (magnesia), calcium oxide, strontium oxide, and mixtures of any two or more these metal oxides. A representative mixture is a mixture of silica and alumina. Otherwise, as opposed to such mixture, the dehydrogenation solid support may comprise an alumino silicate, such as a zeolite, for example having any of the structure types described herein. In the case of an aluminosilicate or zeolite, with respect to types of dehydrogenation solid supports, these may be considered types of metal oxides for purposes of this disclosure. Therefore, for example, the dehydrogenation solid support may comprise a dehydrogenation support metal oxide, a dehydrogenation support zeolite, or a combination thereof. Preferred metal oxides for use in the dehydrogenation solid support are those, such as silica, having low acidity and therefore low cracking activity. According to other embodiments, the dehydrogenation solid support may comprise a stannosilicate as described herein, for example in an amount from about 20 wt-% to about 100 wt-%, from about 50 wt-% to about 100 wt-%, or from about 75 wt-% to about 100 wt-%, relative to the total weight of the dehydrogenation solid support, excluding the weight of the dehydrogenation active metal(s). In some cases, the dehydrogenation solid support may comprise substantially all (*e.g.,* greater than about 95 wt-%) of a stannosilicate. In further embodiments, dehydrogenation catalyst may comprise a stannosilicate as described herein, for example in an amount from about 20 wt-% to about 99 wt-%, from about 50 wt-% to about 98 wt-%, or from about 75 wt-% to about 98 wt-%, relative to the catalyst weight.

According to particular embodiments, the dehydrogenation solid support or base material may comprise a metal oxide that is present in a mixed metal oxide, together with at least one further dehydrogenation support metal oxide (as a solid support constituent), at various weight ratios. For example, a representative dehydrogenation solid support mixed metal oxide may be an oxide of a first metal selected from the group consisting of Li, Mg, Zn, Fe, Ca, Ni, Co, Mn, and Cu, and the further dehydrogenation support metal oxide may be an oxide of a second metal selected from the group consisting of Al, Ga, Y, In, Fe, Co, Ni, Mn, Cr, Ti, V, Zr, and La. In a specific embodiment, the dehydrogenation solid support may comprise a dehydrogenation solid support mixed metal oxide of magnesium oxide (as a metal oxide of the dehydrogenation solid support) and aluminum oxide (as a further dehydrogenation solid support metal oxide) or otherwise a dehydrogenation solid support mixed metal oxide of calcium oxide and aluminum oxide. These may be present in the dehydrogenation solid support, or in the dehydrogenation catalyst, in a magnesium oxide : aluminum oxide weight ratio, or a calcium oxide : aluminum oxide weight ratio, from about 1:1 to about 5:1 or from about 1:1 to about 3:1, such as about 3:1. A dehydrogenation solid support mixed metal oxide may be present as, or derived from, a layered double hydroxide (LDH) as described above, such as a magnesium-aluminum LDH or a calcium-aluminum LDH, in which the first and second metals M and M', of the general LDH formula (I) above are Mg and Al, respectively, or otherwise Ca and Al, respectively. Alternatively, or in combination (*e.g.,* as a further support constituent), the dehydrogenation solid support may comprise aluminum oxide (alumina), silicon oxide (silica) or a mixture thereof. According to a specific embodiment, the dehydrogenation solid support may comprise (i) a dehydrogenation solid support mixed metal oxide present as, or derived from, a layered double hydroxide (LDH) and/or (ii) aluminum oxide (alumina), silicon oxide (silica) or a mixture thereof, onto which a dehydrogenation active catalytic component (*e.g.,* comprising one or more dehydrogenation active metals), as described herein, is dispersed. For example, the dehydrogenation solid support may be prepared by physically mixing (i) and/or (ii), preferably wherein (i) is a dehydrogenation solid support mixed metal oxide that is derived from an LDH. Whether or not a metal oxide of a dehydrogenation solid support is present in a mixed metal oxide, this dehydrogenation solid support metal oxide is preferably calcined, meaning that it has been subjected to high temperature (*e.g.,* from 200°C to 800°C) to improve dispersion of the dehydrogenation active catalytic component, change structural characteristics of this dehydrogenation solid support metal oxide, and/or volatilize undesired impurities. Calcination may involve subjecting a metal oxide of the dehydrogenation solid support to an oxidizing heat treatment, for example under a stream of dry air, at a temperature below the sintering temperature of the support (such as more preferably from about 500°C to about 650°C), and for a duration sufficient to eliminate carbon dioxide, for example from 0.1 to 48 hours. The calcination may be conducted at atmospheric pressure or otherwise under elevated pressure or subatmospheric pressure.

In addition to the dehydrogenation solid support or base material, the dehydrogenation catalyst may also comprise a dehydrogenation active catalytic component, such as one or more dehydrogenation active metals (metallic elements having activity for catalyzing dehydrogenation reactions such as reaction 1 above) dispersed on the dehydrogenation solid support as described herein. For example, according to an evaporative impregnation method, a precursor compound of a dehydrogenation active metal is dissolved in solution and contacted with the dehydrogenation solid support *(e.g.,* a metal oxide of this solid support, which may be present in a mixed metal oxide). The dehydrogenation active metal then becomes deposited on the solid support after evaporation of the impregnation solution. Representative dehydrogenation active metals are selected from the group consisting of metals in Group 11, Group 13, and Group 14 of the periodic table, and combinations thereof. For example, the dehydrogenation active metal(s) of the dehydrogenation active catalytic component may be selected from the group consisting of Sn, In, Pb, Ge, Ga, Tl, Cu, Ag, and Au, with Sn, In, Ga, Cu, Ag, and Au, and combinations thereof being exemplary. A preferred dehydrogenation active metal is tin. One or more (*e.g.,* two, three, or four) of these dehydrogenation active metals may be present in the dehydrogenation solid support, such as dispersed on the solid support and/or chemically incorporated into the solid support, as described herein, with this solid support serving as a base material for the dehydrogenation active metal(s). For example, at least a portion of any given dehydrogenation active metal may be dispersed, and at least a portion may be chemically incorporated. In the case of being dispersed, the metal(s) may be dispersed uniformly or non-uniformly in the support, in various oxidation states, including their zero valence or elemental metal form. A given dehydrogenation active metal may alternatively be present at other oxidation states, such as its oxide state (*e.g.,* as tin oxide), or otherwise in more than a single oxidation state (*e.g.,* in a mixed oxidation state). The one or more dehydrogenation active metals, or compounds (*e.g.,* oxides) of such metals, may each be present independently in an amount, or may otherwise be present in a combined amount, generally from about 0.1 wt-% to about 25 wt-%, such as from about 0.1 wt-% to about 6 wt-%, or generally from about 1 wt-% to about 25 wt-%, typically from about 3 wt-% to about 25 wt-%, and often from about 3 wt-% to about 18 wt-%, by weight of the dehydrogenation solid support or base material, or otherwise by weight of the dehydrogenation catalyst. For example, the one or more dehydrogenation active metals may each be present independently (*i.e*., separately and independently of one another) in an amount generally from about 0.1 wt-% to about 25 wt-%, such as from about 0.1 wt-% to about 6 wt-% or from about 0.1 wt-% to about 3 wt-%, or generally from about 1 wt-% to about 25 wt-%, typically from about 3 wt-% to about 21 wt-%, and often from about 3 wt-% to about 16 wt-%, by weight of the dehydrogenation solid support or base material, or otherwise by weight of the dehydrogenation catalyst. Therefore, according to more specific embodiments, tin may be present in the dehydrogenation catalyst in an amount from about 3 wt-% to about 15 wt-%, such as from about 3 wt-% to about 10 wt-%, and one or more additional metals selected from the group consisting of In, Pb, Ge, Ga, Tl, Cu, Ag, and Au may each additionally be present independently in an amount, or may otherwise be present in a combined amount, from about 0.1 wt-% to about 6 wt-%, such as from about 0.1 wt-% to about 3 wt-%. In such specific embodiments, or more generally in embodiments in which tin is a dehydrogenation active metal, all or at least a portion of the tin may be chemically incorporated into the dehydrogenation solid support and all or at least a portion of the one or more additional metals may be dispersed on the support. The degree or extent of chemical incorporation of the tin may be as described above with respect to mesoporous stannosilicates, with such degree of extent being at least a portion (*e.g.,* at least about 10% or at least about 25%), a predominant amount (*e.g.,* at least about 50% or at least about 80%), or substantially all (*e.g.,* at least about 95%). In addition to one or more Group 11, Group 13, and/or Group 14 dehydrogenation active metals, one or more other dehydrogenation active metals, including metals that may promote the activity of other metals (*i.e*., promoter metals), may also be present in the dehydrogenation catalyst, for example in an amount or combined amount from about 0.1 wt-% to about 3 wt-%, such as from about 0.5 wt-% to about 2 wt-%, by weight of the dehydrogenation solid support or base material, or otherwise by weight of the dehydrogenation catalyst. Such one or more other dehydrogenation active metals may be selected from the group consisting of elements in Groups 6-10 of the periodic table (*e.g.,* Pt, Mo, Ru, Os, and/or Ir) and/or elements in Group 1 and Group 2 of the periodic table, namely alkali metals and/or alkaline earth metals (*e.g.,* Li, Na, K, Rb, Cs, Be, Mg, Ca, Sr, and/or Ba).

Whereas the dehydrogenation active metal(s) may be dispersed on the dehydrogenation solid support or base material by impregnation (*e.g.,* evaporative impregnation), according to other embodiments, the dehydrogenation solid support or base material may be in the form of a zeolite or other metal oxide (*e.g.,* silica, such as mesoporous silica, or alumina) that serves as a framework for chemical incorporation or bonding of any of the dehydrogenation active metals as described above. The base material may therefore be in the form of such framework, including a silicate or aluminosilicate framework, for example a zeolite framework having zeolite structure type as described and referred to in Baerlocher Ch., McCusker L.B., and Olson, D.H., Atlas of Zeolite Framework Types, 6th Revised Ed., Elsevier (2007), in which a portion of the silicon (Si) atoms of tetrahedral silicates (SiO₄), or other tetrahedral lattice sites, are substituted by atoms of the dehydrogenation active metal(s), for example substituted by tin (Sn) atoms. In view of the present disclosure, including the disclosed synthesis methods, it can be appreciated that the chemical incorporation or bonding of a dehydrogenation active metal into a framework (network or matrix), such as chemical incorporation or bonding with the base material, differs from the deposition of such metal onto, or even within, a porous solid support, for example using impregnation methods. The difference in incorporation or bonding, as opposed to deposition, resides in the dehydrogenation active metal (*e.g.,* Sn) being chemically (*e.g.,* covalently) bonded, for example, through intervening oxygen atoms, to other metal atoms (*e.g.,* Si and/or Al) of the base material (*e.g.,* in the form of a silicate or aluminosilicate framework, such as comprising a zeolite having a structure type as described herein), resulting in specific electronic characteristics and distribution profiles. Bonding of Sn through intervening oxygen atoms can result in the incorporation of Sn into the crystal lattice of the base material, whereas the formation of "polymeric" tin or tin oxide, *i.e.,* by the formation of -Sn-Sn- bonds or -Sn-O-Sn- bonds, can result in incorporation of Sn into the base material, although not necessarily within its crystal lattice (as crystalline SnO₂ or Sn). The chemical incorporation or bonding of one or more dehydrogenation active metals into a framework, such as in the case of a mesoporous stannosilicate, can be verified by hydrogen-temperature programmed reduction (H₂-TPR) and other techniques to confirm the nature of atoms, and/or distribution of such atoms, within a crystalline structure. This incorporation may be achieved using synthesis methods utilizing precipitation as described herein.

Regardless of the particular manner in which the dehydrogenation active metal(s) is/are supported on the dehydrogenation solid support, such as by impregnation and/or chemical incorporation into a framework, such dehydrogenation active metals (Group 11, Group 13 and/or Group 14 metals), as well as other metals, such as promoter metal(s), as described above, may be present in amounts or combined amounts according to any of the embodiments described above.

A base material, such as in the form of a framework as described above and having incorporated therein one or more dehydrogenation active metals, is mesoporous, for example such that the dehydrogenation solid support has a pore size, or average pore size, in the range of 2 nanometers (nm) to 50 nm. Preferably, in the case of a mesoporous metal oxide that serves as a framework for incorporation of dehydrogenation active metal(s), at least about 80%, or at least about 90%, of the pore volume of the dehydrogenation solid support can be attributed to pores within this size range. An exemplary dehydrogenation catalyst having such pore size characteristics is a mesoporous stannosilicate, in which Sn atoms are substituted into a metal oxide framework, such as a zeolite framework of zeolite structure type as described above, or a framework of a mesoporous silica such as SBA-15 or MCM-41 mesoporous silica. In the case of a dehydrogenation solid support or base material comprising a metal oxide that serves as a framework for incorporation of any one or more dehydrogenation active metals described above, such dehydrogenation active metal(s) may be present in an amount, or combined amount as described above with respect to such metal(s) being dispersed on the solid support (*e.g.,* by evaporative impregnation), namely from about 0.1 wt-% to about 25 wt-%, such as from about 0.1 wt-% to about 6 wt-%, or generally from about 1 wt-% to about 25 wt-%, typically from about 3 wt-% to about 25 wt-%, and often from about 3 wt-% to about 18 wt-%, by weight of the dehydrogenation solid support or base material, or otherwise by weight of the dehydrogenation catalyst. That is, all of the dehydrogenation active metal(s), or at least that/those portion(s) of the dehydrogenation active metal(s) that are incorporated into the framework (*i.e.*, according to embodiments in which the given amounts do not preclude the presence of additional amounts that are not incorporated into a framework), are present in such amounts. For example, tin (Sn) may be present in the solid support or base material, or in the catalyst as a whole, in such amounts (*e.g.,* from about 3 wt-% to about 15 wt-%), with the tin being incorporated into a framework. According to a specific embodiment, tin may be incorporated into a metal oxide framework and present in the dehydrogenation catalyst, for example in an amount from about 3 wt-% to about 15 wt-%, and one or more additional metals selected from the group consisting of In, Pb, Ge, Ga, Tl, Cu, Ag, Au may additionally be present, for example in an amount, or combined amount, from about 0.1 wt-% to about 6 wt-% by weight of the dehydrogenation solid support or base material, or otherwise by weight of the dehydrogenation catalyst. Unlike tin, the one or more additional metals may be excluded from being incorporated into the framework, such as in the case of being deposited onto the surface of, or otherwise external to the pores of, the dehydrogenation solid support. Particular examples of dehydrogenation catalysts are mesoporous stannosilicates having tin incorporated into a base silicate framework (*i.e*., with silicon atoms of tetrahedral silicate lattice sites being substituted with tin), with this tin being present in an amount of at least about 3 wt-%, such as from about 3 wt-% to about 15 wt-%, or from about 3 wt-% to about 10 wt-%, by weight of the dehydrogenation solid support or base material, or otherwise by weight of the dehydrogenation catalyst.

In general, in the case of a dehydrogenation solid support or base material that comprises a metal oxide that serves as a framework for incorporation of any of the dehydrogenation active metals, or otherwise any other solid support as described herein (*e.g.,* a metal oxide, which may optionally be present in a mixed metal oxide), such support may have one or more of any of the dehydrogenation active metals, and/or promoter metals, described above deposited on such support (*e.g.,* on its surface), or at least deposited external to pores of such support. Such metal(s) may also be deposited within the pores of such support. Regardless of the particular manner in which the dehydrogenation active metal(s) is/are supported on the dehydrogenation solid support, such as by impregnation and/or chemical incorporation into a framework, the dehydrogenation solid support or base material and any dehydrogenation active metal (*e.g*., Group 11, Group 13, and/or Group 14 metals, such as Sn) or combination of such metals, as described above, whether or not incorporated into a framework, may be present in a combined amount of generally at least about 90 wt-% by weight, typically at least about 95 wt-% by weight, and often at least about 99 wt-%, by weight of the dehydrogenation catalyst. In embodiments in which a solid binder is a component of the dehydrogenation catalyst, the binder may be considered, for purposes of determining the combined amount dehydrogenation active metal(s) and dehydrogenation solid support, as contributing to the weight of the dehydrogenation solid support. In some cases, the dehydrogenation solid support or base material (including any optional binder) and dehydrogenation active metal(s) may be present in a combined amount of at least about 99.9 wt-%, by weight of the dehydrogenation catalyst. That is, components other than those of the dehydrogenation solid support or base material and the dehydrogenation active metal(s), in any of the embodiments described above, may be substantially absent. In this regard, particular aspects of the invention are associated with the discovery of catalysts that are effective for paraffin dehydrogenation, but that advantageously lack any significant amounts of precious metals (*e.g.,* Pt) and/or hazardous metals (*e.g.,* Cr). According to preferred embodiments, a combined amount, by weight of the dehydrogenation solid support or base material, or otherwise by weight of the dehydrogenation catalyst, of chromium and/or metals in Groups 8-10 of the periodic table, is less than about 0.1 wt-%, or even less than about 0.01 wt-%.

### Metathesis Catalysts

Representative metathesis catalysts comprise a metathesis active catalytic component (*e.g.,* comprising one or more olefin metathesis active metals) that is supported on a metathesis solid support, which serves as a carrier of this component. For example, the metathesis active catalytic component may be dispersed uniformly or possibly non-uniformly (*e.g.,* preferentially near the outer surface) within the metathesis solid support, and optional other support constituents. The metathesis solid support may comprise a metal oxide such as aluminum oxide (alumina), silicon oxide (silica), zirconium oxide (zirconia), titanium oxide (titania), magnesium oxide, calcium oxide, strontium oxide, and mixtures of any two or more these metal oxides. Preferred metal oxides are aluminum oxide (alumina), silicon oxide (silica), zirconium oxide (zirconia), titanium oxide (titania), and mixtures thereof. A representative mixture is a mixture of silica and alumina. Otherwise, as opposed to such mixture, the metathesis solid support may comprise an aluminosilicate, such as a zeolite, for example having any of the structure types described herein. In the case of an aluminosilicate or zeolite, with respect to types of metathesis solid supports, these may be considered types of metal oxides for purposes of this disclosure. In a preferred embodiment, the metathesis solid support comprises a metal oxide independently be selected from magnesium oxide, calcium oxide, silicon oxide (silica), strontium oxide, or other metal oxide having low acidity and therefore low cracking activity. The metathesis solid support may additionally, or otherwise, comprise aluminum oxide. According to some embodiments, the metal oxide of the metathesis solid support, such as magnesium oxide or calcium oxide, may be present in a mixed metal oxide, for example together with aluminum oxide as a magnesium-aluminum mixed metal oxide or a calcium aluminum mixed metal oxide.

More generally, a metal oxide of the metathesis solid support may be present in a mixed metal oxide, together with at least one further metathesis solid support metal oxide (as a support constituent), at various weight ratios. For example, a representative metathesis solid support mixed metal oxide may be an oxide of a first metal selected from the group consisting of Li, Mg, Zn, Fe, Ca, Ni, Co, Mn, and Cu, and the further metathesis solid support metal oxide may be an oxide of a second metal selected from the group consisting of Al, Ga, Y, In, Fe, Co, Ni, Mn, Cr, Ti, V, Zr, and La. In a specific embodiment, the metathesis catalyst may comprise a metathesis solid support mixed metal oxide of magnesium oxide (as the metathesis solid support metal oxide) and aluminum oxide (as the further metathesis solid support metal oxide), or otherwise a metathesis solid support mixed metal oxide of calcium oxide and aluminum oxide. These may be present in the mixed metal oxide, or in the metathesis solid support, in a magnesium oxide : aluminum oxide weight ratio, or otherwise a calcium oxide : aluminum oxide weight ratio, from about 1:10 to about 10:1, or from about 1:8 to about 1:2, such as about 1:5, or otherwise a weight ratio from about 1:1 to about 5:1 or from about 1:1 to about 3:1, such as about 3:1. Any mixed metal oxides may be present as, or derived from, a layered double hydroxide (LDH) as described above with respect to the dehydrogenation solid support, with a magnesium-aluminum LDH or a calcium-aluminum LDH being representative. Alternatively, or in combination (*e.g.,* as a further solid support constituent), the metathesis catalyst may comprise aluminum oxide (alumina), silicon oxide (silica) or a mixture thereof. According to a specific example, the metathesis catalyst may comprise both a metathesis solid support mixed metal oxide, such as one prepared as, or derived from, an LDH (*e.g.,* a magnesium-aluminum LDH or a calcium-aluminum LDH) and further comprise another metathesis solid support metal oxide such as silicon oxide. According to a specific embodiment, the metathesis catalyst may comprise (i) a metathesis solid support mixed metal oxide that is, or that is derived from, a layered double hydroxide (LDH) and (ii) aluminum oxide (alumina), silicon oxide (silica) or a mixture thereof, with both (i) and (ii), or otherwise with only (i) or only (ii), having a metathesis active catalytic component, as described herein, deposited thereon. For example, in the case of the olefin metathesis active metal being deposited onto only (ii) and not (i), the metathesis catalyst may be prepared by depositing the olefin metathesis active metal onto (ii), for example by impregnation with a solution of a precursor compound of the olefin metathesis active metal, and then physically mixing (i) with (ii) having the olefin metathesis active metal deposited thereon, preferably wherein (i) is a metathesis solid support mixed metal oxide that is derived from an LDH. The resulting mixture of (i) and (ii) may then be calcined as described herein. In general, therefore, either or both of the support constituents (i) and (ii) may be calcined. The support constituents (i) and/or (ii), onto which the one or more olefin metathesis active metals are dispersed, may or may not be the same constituents that are calcined. Whether or not a metathesis solid support metal oxide is present in, or comprises, a mixed metal oxide, this support metal oxide is preferably calcined, meaning that it has been subjected to conditions as described above with respect to the dehydrogenation solid support metal oxide, to improve dispersion of the olefin metathesis active catalytic component, change structural characteristics of this solid support metal oxide, and/or volatilize undesired impurities.

The metathesis catalyst may further comprise, as further solid support constituents, (i) one or more other metathesis solid support metal oxides and/or (ii) one or more zeolites. Representative zeolites have a structure type selected from the group consisting of FAU, FER, MEL, MTW, MWW, MOR, BEA, LTL, MFI, LTA, EMT, ERI, MAZ, MEI, SBA-15, MCM, and TON, and preferably selected from one or more of FAU, FER, MWW, MOR, BEA, LTL, MFI, SBA-15, and MCM. As noted above, the structures of zeolites having these and other structure types are described, and further references are provided, in Baerlocher Ch., McCusker L.B., and Olson, D.H., Atlas of Zeolite Framework Types, 6th Revised Ed., Elsevier (2007). Specific examples include zeolite Y (FAU structure), zeolite X (FAU structure), MCM-22 (MWW structure), and ZSM-5 (MFI structure), with zeolite Y and ZSM-5 being preferred. Zeolites may be used in any of their ion (or ion exchange) forms, such as their alkali or alkaline earth metal forms (*e.g.,* sodium form) or their hydrogen forms. In the case of zeolite Y, the hydrogen form of this zeolite (HY zeolite) is a preferred form. According to a specific embodiment, the metathesis catalyst may comprise silica and a zeolite, such as zeolite Y (*e.g.,* HY zeolite) or ZSM-5.

In addition to a metathesis solid support metal oxide, the metathesis catalyst may also comprise an olefin metathesis active catalytic component, such as one or more olefin metathesis active metals (metallic elements having activity for catalyzing metathesis reactions such as reaction 2 above) dispersed on the metathesis solid support, such as a metal oxide as described herein. For example, an evaporative impregnation method as described above with respect to preparation of the dehydrogenation catalyst may be used, but instead with a precursor compound of an olefin metathesis active metal. Representative olefin metathesis active metals may include any one or more of those metals in Group 6 and Group 7 of the periodic table (*e.g.,* tungsten). Preferred olefin metathesis active metals are selected from the group consisting of tungsten (W), molybdenum (Mo), rhenium (Re), and combinations thereof. One or more of these olefin metathesis active metals may be present in the metathesis solid support, such as dispersed uniformly or non-uniformly therein, in various oxidation states, including their zero valence or elemental metal form. A given olefin metathesis active metal may alternatively be present at other oxidation states, such as its oxide state, or otherwise in more than a single oxidation state (*e.g.,* in a mixed oxidation state). The one or more olefin metathesis active metals, or compounds (*e.g.,* oxides) of such metals, may be present in an amount, or in a combined amount, generally from about 1% to about 15%, typically from about 2% to about 12%, and often from about 4% to about 12%, by weight of the metathesis solid support, or otherwise by weight of the metathesis catalyst.

Particular examples of metathesis catalysts comprise one or more of tungsten, molybdenum, and/or rhenium as olefin metathesis active metals in their oxide state (*i.e.,* as tungsten oxide or WO₃, as molybdenum oxide or MoO₃, and/or as rhenium oxide or Re₂O₇) and further comprise a metathesis solid support, as described herein. According to specific embodiments, the metathesis catalyst comprises (i) one or more of tungsten, molybdenum, and/or rhenium as olefin metathesis active metals in an amount or combined amount as described above, and (ii) one or more metathesis solid support metal oxides (*e.g.,* silica), wherein (i) and (ii) are present in a combined amount representing substantially all or all (*e.g.,* greater than about 90%, greater than about 95%, or greater than about 99%) of the weight of the metathesis catalyst. According to other specific embodiments the metathesis catalyst comprises (i) one or more of tungsten, molybdenum, and/or rhenium as olefin metathesis active metals in an amount or combined amount as described above, (ii) one or more metathesis solid support metal oxides (*e.g.,* silica), and (iii) one or more zeolites (*e.g.,* zeolite Y), wherein (i), (ii), and (iii) are present in a combined amount representing substantially all or all (*e.g.,* greater than about 90%, greater than about 95%, or greater than about 99%) of the weight of the metathesis catalyst. The one or more zeolites may be present in an amount generally from about 0.1 to about 60%, typically from about 0.5 to about 30%, and often from about 1 to about 20%, by weight of the metathesis catalyst.

Other particular examples of metathesis catalysts comprise one or more of tungsten, molybdenum, and/or rhenium as olefin metathesis active metals in their oxide state (*i.e.,* as tungsten oxide or WO₃, as molybdenum oxide or MoO₃, and/or as rhenium oxide or Re₂O₇) and further comprise a metathesis solid support mixed metal oxide comprising (or present as) an LDH, such as a magnesium-aluminum LDH or otherwise a calcium-aluminum LDH, in which the first and second metals, M and M', of the general LDH formula (I) above are Mg and Al or otherwise Ca and Al. A specific metathesis catalyst, for example, comprises tungsten oxide at a weight percent within the ranges described above with respect to compounds of olefin metathesis active metals (*e.g.,* from about 4% to about 8% by weight, relative to the weight of the metathesis catalyst) deposited on a metathesis solid support, which may comprise a mixed metal oxide comprising an LDH (*e.g.,* a magnesium-aluminum LDH or a calcium-aluminum LDH). The olefin metathesis active metal(s), according to some embodiments, may be deposited predominantly (*e.g.,* to an extent of at least about 50% or to an extent of at least about 80%) or substantially all (*e.g.,* to an extent of at least about 95%) on support constituents other than an LDH, such as being deposited predominantly or substantially on another metal oxide (*e.g.,* silica) and/or a zeolite (*e.g.,* zeolite Y). The LDH may be present in an amount from generally from about 0.1 to about 80%, typically from about 0.5 to about 50%, and often from about 1 to about 30%, by weight of the metathesis catalyst. The zeolite may be present in an amount generally from about 0.1 to about 60%, typically from about 0.5 to about 30%, and often from about 1 to about 20%, by weight of the metathesis catalyst. Any other metal oxide(s) (*e.g.,* silica) may be present in an amount representing the balance of the weight of the metathesis catalyst, such that (i) the one or more olefin metathesis active metals, or compounds (*e.g.,* oxides) of such metals, (ii) the LDH, (iii) the other metal oxide(s), and (iv) the zeolite are present in a combined amount representing substantially all or all (*e.g.,* greater than about 90%, greater than about 95%, or greater than about 99%) of the weight of the metathesis catalyst. The metathesis solid support mixed metal oxide, in addition to other metathesis support constituents, including other metal oxide(s) (*e.g.,* silica) and/or zeolite(s) (*e.g.,* zeolite Y), may be calcined as described above. According to a particular metathesis catalyst comprising (i), (ii), (iii), and (iv) above, the one or more olefin metathesis active metals (*e.g.,* tungsten present as WO₃) may be dispersed on any of, or any combination of, (ii), (iii), and (iv). Also, any of, or any combination of, the support constituents (ii), (iii), and (iv) may be calcined. The support constituents (ii), (iii), and/or (iv), onto which the one or more olefin metathesis active metals are dispersed, may or may not be the same constituents that are calcined. For example, the one or more olefin metathesis active metals may be dispersed on (iii) and (iv), and not dispersed on (ii), and each of (ii), (iii), and (iv) may be calcined. In a specific example of preparing a metathesis catalyst, (iii) and (iv) may be mixed, such as in the case of preparing a physical mixture of silica and zeolite Y. The one or more olefin metathesis active metals may then be deposited onto the mixture of (iii) and (iv), for example by impregnation with a solution of a precursor compound of the olefin metathesis active metal. The resulting mixture of (iii) and (iv) (*e.g.,* a mixture of silica and zeolite Y), having one or more olefin metathesis active metals deposited thereon, may then be calcined as described herein, optionally following drying. The resulting, calcined mixture of (iii) and (iv) (*e.g.,* a mixture of silica and zeolite Y), having one or more olefin metathesis active metals deposited thereon, optionally following drying, may then be mixed with a mixed metal oxide, for example in the form of a layered double oxide (LDO) that is obtained by thermal treatment (*e.g.,* calcination) of (ii). For example, a mixture of silica and zeolite Y, having one or more olefin metathesis active metals (*e.g.,* tungsten) deposited thereon, may be further mixed with the LDO, such as a magnesium-aluminum LDO or a calcium-aluminum LDO, obtained by calcination of the corresponding LDH. The further resulting mixture may then provide a representative metathesis catalyst, comprising the one or more olefin metathesis active metals (*e.g.,* tungsten present as WO₃) dispersed on a mixture of one or more metal oxides (*e.g.,* silica) and a zeolite (*e.g.,* zeolite Y), with the metathesis catalyst further comprising a mixed metal oxide (*e.g.,* a magnesium-aluminum mixed metal oxide or a calcium-aluminum mixed metal oxide, resulting from calcination of a magnesium-aluminum LDH or a calcium-aluminum LDH).

According to particular embodiments of the invention, the dehydrogenation catalyst and/or the metathesis catalyst may have a substantially uniform or homogeneous composition. According to other embodiments, the dehydrogenation catalyst and/or metathesis catalyst may have a non-uniform composition, for example with respect to a radial concentration profile of the dehydrogenation active metal(s) and/or the olefin metathesis active metal(s). For example, such metals may be more concentrated in the interiors of catalyst particles and may be less concentrated, or even absent, at or near the surfaces of catalyst particles. Otherwise, such metals may be more concentrated at or near the surfaces of catalyst particles and may be less concentrated, or even absent, in the interiors of catalyst particles.

### Dehydrogenation and Metathesis Catalysts

According to yet further embodiments, a representative catalyst system may comprise, or consist of, one catalyst, *i.e.,* a catalyst of a single composition, having both (i) one or more dehydrogenation active metals as described herein, and (ii) one or more olefin metathesis active metals as described herein, disposed on (or present in) a dehydrogenation solid support as described above. For example, at least a portion of any of the dehydrogenation active metal(s) and/or olefin metathesis active metal(s) may be dispersed on any dehydrogenation solid support as described herein by impregnation (*e.g.,* evaporative impregnation). Such dehydrogenation solid supports may comprise metal oxides (*e.g.,* silica and/or alumina), aluminosilicates (*e.g.,* zeolites having any of the structure types described herein), mixed metal oxides, and/or layered double hydroxides, including any of the general and specific types of dehydrogenation solid supports described herein. Otherwise, the dehydrogenation solid support, serving as a support for a single dehydrogenation and metathesis catalyst (*i.e*., as the dehydrogenation and metathesis solid support), may comprise a metal oxide that serves as a framework for incorporation of any of the dehydrogenation active metals as described above. In this case, the chemical incorporation of at least a portion of any such metal(s) (*e.g.,* Sn) may be obtained using a precipitation method as described herein. According to a particular embodiment, the support may be, or may comprise, a stannosilicate (*e.g.,* a microporous stannosilicate, a mesoporous stannosilicate, or a macroporous stannosilicate, as described above), having the dehydrogenation active metal (*e.g.,* tin) being incorporated into the framework of the support. Therefore, a representative dehydrogenation and metathesis catalyst may comprise a stannosilicate support (*e.g.,* a mesoporous stannosilicate support), having tin incorporated into a silicate framework (in which tetrahedral Si is substituted by Sn), and one or more metals supported on the mesoporous stannosilicate support and selected from the group consisting of metals in Group 6 and Group 7 of the periodic table. The dehydrogenation and metathesis solid support, or dehydrogenation and metathesis catalyst, may comprise such stannosilicate in any of the amounts as described above with respect to the dehydrogenation solid support, or dehydrogenation catalyst. In the case of dehydrogenation catalysts comprising tin, this dehydrogenation active metal may be chemically incorporated into a stannosilicate or other dehydrogenation solid support to an extent or degree as described above.

Regardless of the particular manner in which the dehydrogenation active metal(s) and olefin metathesis active metal(s) are present in the dehydrogenation solid support, such as by impregnation and/or chemical incorporation into a framework, such dehydrogenation active metals (Group 11, Group 13, and/or Group 14 metals), as well as other metals, such as promoter metal(s), as described above, may be present in amounts or combined amounts as described above and pertaining to dehydrogenation catalysts. Likewise, the olefin metathesis active metal(s) (*e.g.,* selected from Group 6 and Group 7 of the periodic table), may be present in amounts as described above and pertaining to metathesis catalysts. For example, the tin may be present in the dehydrogenation and metathesis catalyst in an amount from about 0.02 wt-% to about 30 wt-% and/or the metal(s) selected from Group 6 and Group 7 of the periodic table may be present in the dehydrogenation and metathesis catalyst in an amount, or combined amount, from about 0.05 wt-% to about 15 wt-%. Regardless of the particular manner in which the dehydrogenation active metal(s) and olefin metathesis active metal(s) are present in the dehydrogenation solid support, suitable dehydrogenation and metathesis catalysts may be prepared by first preparing a dehydrogenation catalyst according to any of the general and specific preparation procedures described herein, followed by impregnation of such dehydrogenation catalyst with any of the olefin metathesis active metal(s) as described herein. More generally, representative dehydrogenation and metathesis catalysts comprise any dehydrogenation catalyst described herein, having any of the olefin metathesis active metals described herein (*e.g.,* including the particular amounts of such metals described herein) supported on such dehydrogenation catalyst (*e.g.,* dispersed thereon by impregnation).

In other embodiments, however, the dehydrogenation active metal(s) and the olefin metathesis active metal(s) may be present in a dehydrogenation and metathesis catalyst, or in a support for such catalyst, such as dispersed on the solid support and/or chemically incorporated into the solid support, in alternative amounts, relative to those amounts described above and pertaining to dehydrogenation catalysts and metathesis catalysts individually. Such lower amounts may also correspond to those amounts in an overall catalyst system comprising both of these types of catalyst. For example, a dehydrogenation and metathesis catalyst, or otherwise a catalyst system comprising a dehydrogenation catalyst and a metathesis catalyst, may comprise one or more dehydrogenation active metals as described above (*e.g.,* Sn, In, Pb, Ge, Ga, Tl, Cu, Ag, Au) independently in an amount, or otherwise may be present in a combined amount, generally from about 0.1 wt-% to about 20 wt-%, such as from about 0.1 wt-% to about 6 wt-%, or from about 3 wt-% to about 16 wt-% and typically from about 0.5 wt-% to about 15 wt-%, such as from about 0.5 wt-% to about 3 wt-% or from about 5 wt-% to about 10 wt-%, by weight of the catalyst or catalyst system. For example, according to a specific embodiment, tin may be present in the dehydrogenation and metathesis catalyst, or catalyst system, in an amount generally from about 1 wt-% to about 20 wt-%, typically from about 3 wt-% to about 16 wt-%, and often from about 5 wt-% to about 10 wt-%. One or more additional metals selected from the group consisting of In, Pb, Ge, Ga, Tl, Cu, Ag, Au may each additionally be present in the dehydrogenation and metathesis catalyst, or catalyst system, independently in an amount generally from about 0.1 wt-% to about 10 wt-%, typically from about 0.1 wt-% to about 7 wt-%, and often from about 0.1 wt-% to about 6 wt-%. Other dehydrogenation active metals, including metals that may promote the activity of other metals (*i.e.*, promoter metals), may be present in the dehydrogenation and metathesis catalyst, or catalyst system, for example independently in an amount, or otherwise in a combined amount, from about 0.005 wt-% to about 3 wt-%, such as from about 0.01 wt-% to about 0.1 wt-%). Such other metals may be selected from the group consisting of elements in Groups 6-10 of the periodic table (*e.g.,* Pt, Mo, Ru, Os, and/or Ir). Elements in Group 1 and/or Group 2 of the periodic table, namely alkali metals and/or alkaline earth metals (*e.g.,* Li, Na, K, Rb, Cs, Be, Mg, Ca, Sr, and/or Ba) may be present in the dehydrogenation and metathesis catalyst, or catalyst system, for example in an amount, or combined amount from about 0.001 wt-% to about 0.1 wt-%, such as from about 0.005 wt-% to about 0.03 wt-%. Alternatively, or in combination, a dehydrogenation and metathesis catalyst, or otherwise a catalyst system comprising a dehydrogenation catalyst and a metathesis catalyst, may comprise one or more olefin metathesis active metals as described above (*e.g.,* selected from Group 6 and Group 7 of the periodic table, such as W) independently in an amount, or otherwise in a combined amount, generally from about 0.05% to about 15%, typically from about 0.1% to about 12%, and often from about 2% to about 8%, by weight of the catalyst or catalyst system.

### Catalyst Systems

Representative catalyst systems comprise both a dehydrogenation catalyst and a metathesis catalyst as described herein, with such catalysts being present in one or more beds, for example comprising the catalyst(s) in isolation or otherwise mixed (*e.g*., physically mixed). A catalyst "bed" (*e.g.,* fixed bed) of a system refers to a group of catalyst particles within a contiguous volume and having a constant or substantially constant (uniform or substantially uniform) composition. Different beds may or may not be separated, such as by a relatively inert spacing material (*e.g.,* alumina spheres). For example, a catalyst bed may comprise (i) all or substantially all *(e.g.,* greater than about 90% by weight, greater than about 95% by weight, or greater than about 99% by weight) of a dehydrogenation catalyst as described herein, (ii) all or substantially all (*e.g.,* greater than about 90% by weight, greater than about 95% by weight, or greater than about 99% by weight) of a metathesis catalyst as described herein, or (iii) a mixture (*e.g.,* a uniform physical mixture) of a dehydrogenation catalyst as described herein and a metathesis catalyst as described herein, at a given mixing ratio. The mixing weight ratio of dehydrogenation catalyst : metathesis catalyst may be, for example, from about 0.10:1 to about 10:1, such as from about 0.10:1 to about 4:1, from about 0.25:1 to about 3:1, from about 0.25:1 to about 2:1, from about 0.25:1 to about 1:1, or from about 0.25:1 to about 0.5:1.

It is noted that, with respect to the description of various beds of catalyst systems herein, references to "a physical mixture" or "a mixture" of a dehydrogenation catalyst (or particles of such catalyst) and a metathesis catalyst (or particles of such catalyst) are meant to likewise disclose embodiments in which such "physical mixture" or "mixture" is replaced by a single composition of a dehydrogenation and metathesis catalyst as described above.

A first type of catalyst system may be a single bed of a physical mixture of these catalysts, or multiple beds (*e.g.,* 2 or more, such as from 2 to 10, or 3 or more, such as from 3 to 6) of physical mixtures at differing mixing weight ratios, for example within those mixing weight ratios described above. In a specific embodiment, such first type of catalyst system may be a single bed of a physical mixture of a dehydrogenation catalyst and a metathesis catalyst at mixing weight ratio of dehydrogenation catalyst : metathesis catalyst within a range as described above. A second type of catalyst system may be multiple (*e.g.,* 2 or more, such as from 2 to 10, or 3 or more, such as from 3 to 6), alternating, separate beds of all or substantially dehydrogenation catalyst and all or substantially all metathesis catalyst. In the case of beds comprising all or substantially dehydrogenation catalyst, or all or substantially all metathesis catalyst, any two or more of such beds, for example all of such beds of a catalyst system comprising two or more of such beds, may be present in the system in a weight ratio of one or more bed(s) comprising all or substantially dehydrogenation catalyst : one or more bed(s) comprising all or substantially all metathesis catalyst, corresponding to the mixing weight ratios described above. A third type of catalyst system may be (i) one or more beds of a physical mixture of a dehydrogenation catalyst and a metathesis catalyst and (ii) one or more beds of all or substantially dehydrogenation catalyst and/or one or more beds of all or substantially all metathesis catalyst. In this case, the mixing weight ratios described above may apply to (i) mixing weight ratios of any of the one or more beds of a physical mixture, (ii) the weight ratio of one or more bed(s) comprising all or substantially dehydrogenation catalyst : one or more bed(s) comprising all or substantially all metathesis catalyst, (iii) the weight ratio of one or more beds of a physical mixture of a dehydrogenation catalyst and a metathesis catalyst : one or more bed(s) comprising all or substantially dehydrogenation catalyst, and/or (iv) the weight ratio of one or more beds of a physical mixture of a dehydrogenation catalyst and a metathesis catalyst : one or more bed(s) comprising all or substantially metathesis catalyst. In a specific embodiment, such third type of catalyst system may be (i) a single bed of a physical mixture of a dehydrogenation catalyst and a metathesis catalyst at mixing weight ratio of dehydrogenation catalyst : metathesis catalyst from about 0.25:1 to about 1:1, such as from about 0.5:1 to about 1:1 and (ii) a single bed of all or substantially all metathesis catalyst. In another specific embodiment, such third type of catalyst system may be (i) a single bed of a physical mixture of a dehydrogenation catalyst and a metathesis catalyst at mixing weight ratio of dehydrogenation catalyst : metathesis catalyst from about 0.25:1 to about 3:1, such as from about 0.75:1 to about 1.5:1 and (ii) a single bed of all or substantially all dehydrogenation catalyst and a single bed of all or substantially all metathesis catalyst, with a weight ratio of the single bed of all or substantially all dehydrogenation catalyst: single bed of all or substantially all dehydrogenation catalyst being from about 0.25:1 to about 3:1, such as from about 0.75:1 to about 1.5:1.

In other representative embodiments, in an overall catalyst system as described herein, regardless of the configuration of the catalyst beds (*e.g.,* mixed and/or non-mixed dehydrogenation and metathesis catalysts), the dehydrogenation catalyst and olefin metathesis catalyst may be present in a weight ratio corresponding to the mixing ratios described above, *i.e.,* from about 0.10:1 to about 10:1, such as from about 0.10:1 to about 4:1, from about 0.25:1 to about 3:1, or from about 0.25:1 to about 1:1 (*e.g.,* about 0.375:1 or about 1:1). In representative embodiments, the dehydrogenation catalyst particles and the metathesis catalyst particles, and/or optionally the dehydrogenation and metathesis catalyst particles, are present in a combined amount representing all or substantially all (*e.g.,* greater than about 90%, greater than about 95%, or greater than about 99%) of the weight of the catalyst system, or all or substantially all of the weight of at least one bed of such system. That is, preferably few or no types of other catalysts or other materials are present in the catalyst system, or catalyst bed of such system (*e.g.,* catalyst bed that is disposed in a dehydrogenation/metathesis reactor). According to other representative embodiments, the dehydrogenation catalyst particles and the metathesis catalyst particles, and/or optionally the dehydrogenation and metathesis catalyst particles, are present in a combined amount representing less than all or substantially all of the weight of the catalyst system, or less than all or substantially all of the weight of at least one bed of such system, with such combined amount being, for example, in a range from about 25% to about 90%, such as from about 75% to about 90%, from about 55% to about 90%, or from about 35% to about 55%, of the weight of the catalyst system or the weight of at least one bed of such system. In such embodiments, the balance of the weight (*e.g.,* from about 10% to about 75%, such as from about 10% to about 25%, from about 10% to about 45%, or from about 45% to about 65% of the weight) of the catalyst system or at least one bed of such system may include one or more types of other catalysts, such as a cracking catalyst, an alkylation catalyst, or an isomerization catalyst, if adjustments to the product composition are desired, for example in terms of the molecular weight or degree of branching of the hydrocarbon species. In the case of a cracking catalyst, this may comprise a molecular sieve such as a zeolite (*e.g.,* ZSM-5 or ZSM-11), a non-zeolitic molecular sieve (*e.g.,* SAPO-11 or SAPO-34), and/or silicalite. A particular cracking catalyst comprises ZSM-5 having a framework silica to alumina (SiO₂/Al₂O₃) molar ratio from about 20 to about 3000, such as from about 20 to about 1500, from about 40 to about 3000, from about 200 to about 800, or from about 400 to about 1600.

A further representative catalyst system may comprise one or more beds (*e.g.,* a single bed) of a physical mixture of dehydrogenation catalyst and metathesis catalyst, in addition to one or more beds (*e.g.,* a single bed) of all or substantially dehydrogenation catalyst, or all or substantially all metathesis catalyst. According to a specific embodiment, a catalyst system may comprise, or consist of, a first catalyst bed comprising a physical mixture of a dehydrogenation catalyst and metathesis catalyst, in a mixing weight ratio as described above (*e.g.,* about 0.75:1), and a second catalyst bed comprising all or substantially all metathesis catalyst. The first catalyst bed of the physical mixture may be an upstream bed and the second catalyst bed of the metathesis catalyst may be a downstream bed, with the terms "upstream" and "downstream" having the significance, with respect to the direction of the main or bulk flow of the feed, as described below. In the case of a mixing weight ratio of dehydrogenation catalyst : metathesis catalyst of 0.75:1 in the first catalyst bed, for example, in combination with a weight ratio of first catalyst bed : second catalyst bed of 1.75:1, the weight ratio of dehydrogenation catalyst : metathesis catalyst in the system would be 0.375:1. According to another embodiment, a catalyst system may comprise an optional first catalyst bed, followed by two catalyst beds, namely this system may include (i) an optional upstream bed comprising all or substantially all dehydrogenation catalyst particles, (ii) a bed comprising a mixture of dehydrogenation catalyst particles and metathesis catalyst particles in a mixing ratio as described above (*e.g.,* about 0.75:1 or about 1:1), and (iii) a downstream bed comprising all or substantially all metathesis catalyst particles, with the terms "upstream" and "downstream" having the significance, with respect to the direction of the main or bulk flow of the feed, as described below. The weight ratio of (ii) : (iii), or otherwise the weight ratio of (ii) : (i) (if the optional upstream bed is used), may be from 0.5:1 to 10:1, such as from 1:1 to 5:1, or from 1:1 to 3:1, for example in the case of a weight ratio of (ii) : (iii) of 1.75:1 or 1:1 and/or a weight ratio of (ii) : (i) of 1:1. In other embodiments, the weight ratio of (i): (ii) : (iii) may be from about 0.5-1.5 : 0.5-1.5 : 0.5-1.5, with a particular weight ratio being about 1:1:1. In the case of a mixing weight ratio of dehydrogenation catalyst: metathesis catalyst of 1:1 in the bed comprising the mixture, for example, in combination with a weight ratio of (ii) : (iii) of 1:1 and a weight ratio of (i) : (ii) of 1:1, the weight ratio of dehydrogenation catalyst: metathesis catalyst in the system would be 1:1, *i.e.,* 50 wt-% of each catalyst type would be present in the system.

In view of the foregoing description, including the notation that references to "a physical mixture" or "a mixture" of a dehydrogenation catalyst (or particles of such catalyst) and a metathesis catalyst (or particles of such catalyst) are meant to likewise disclose embodiments in which such "physical mixture" or "mixture" is replaced by a single composition of a dehydrogenation and metathesis catalyst, it can be appreciated that further catalyst systems are likewise disclosed. For example, a catalyst system may comprise one or more beds (*e.g.,* a single bed) of a single dehydrogenation and metathesis catalyst, in addition to one or more beds (*e.g.,* a single bed) of all or substantially dehydrogenation catalyst, or all or substantially all metathesis catalyst. According to a specific embodiment, a catalyst system may comprise, or consist of, a first catalyst bed comprising a single dehydrogenation and metathesis catalyst, and a second catalyst bed comprising all or substantially all metathesis catalyst. The first catalyst bed of the single dehydrogenation and metathesis catalyst may be an upstream bed and the second catalyst bed of the metathesis catalyst may be a downstream bed, with the terms "upstream" and "downstream" having the significance, with respect to the direction of the main or bulk flow of the feed, as described below. The weight ratio of the single dehydrogenation and metathesis catalyst: metathesis catalyst in the system may be from about 0.25:1 to about 3:1, such as from about 0.75:1 to about 1.5:1. According to another specific embodiment, a catalyst system may comprise, or consist of, a first catalyst bed comprising all or substantially all dehydrogenation catalyst and a second catalyst bed comprising a single dehydrogenation and metathesis catalyst. The first catalyst bed of the dehydrogenation catalyst may be an upstream bed and the second catalyst bed of the dehydrogenation and metathesis catalyst may be a downstream bed, with the terms "upstream" and "downstream" having the significance, with respect to the direction of the main or bulk flow of the feed, as described below. The weight ratio of the dehydrogenation catalyst : single dehydrogenation and metathesis catalyst in the system may be from about 0.25:1 to about 3:1, such as from about 0.75:1 to about 1.5:1. According to a further specific embodiment, a catalyst system may comprise (i) an optional upstream bed comprising all or substantially all dehydrogenation catalyst particles, (ii) a bed comprising particles of a single dehydrogenation and metathesis catalyst, and (iii) a downstream bed comprising all or substantially all metathesis catalyst particles, with the terms "upstream" and "downstream" having the significance, with respect to the direction of the main or bulk flow of the feed, as described below. The weight ratio of (ii) : (iii), or otherwise the weight ratio of (ii) : (i) (if the optional upstream bed is used), may be from 0.5:1 to 10:1, such as from 1:1 to 5:1, or from 1:1 to 3:1, for example in the case of a weight ratio of (ii) : (iii) of 1.75:1 or 1:1 and/or a weight ratio of (ii) : (i) of 1:1. In other embodiments, the weight ratio of (i) : (ii) : (iii) may be from about 0.5-1.5 : 0.5-1.5 : 0.5-1.5, with a particular weight ratio being about 1:1:1.

In the case of catalyst systems comprising one or more beds (*e.g.,* a single bed) of a physical mixture of dehydrogenation catalyst and metathesis catalyst, in addition to one or more beds (*e.g.,* a single bed) of all or substantially dehydrogenation catalyst, or all or substantially all metathesis catalyst, the same or a different dehydrogenation catalyst, and/or the same or a different metathesis catalyst, may be used in the bed(s) of the physical mixture, as used in the bed(s) of substantially all dehydrogenation catalyst and substantially all metathesis catalyst. Preferably, the same dehydrogenation catalyst and the same metathesis catalyst are used. Therefore, for example, a catalyst system may comprise, or consist of, a bed of a physical mixture dehydrogenation catalyst and metathesis catalyst, in a mixing weight ratio as described above (*e.g.,* 0.375:1), and bed of all or substantially all metathesis catalyst, with the metathesis catalyst being the same as in the physical mixture.

Aspects of the invention relate to performance advantages, in carrying out dehydrogenation and metathesis of a feed comprising a paraffinic hydrocarbon, which are gained from the use of catalysts and catalyst systems as described herein. For example, as described above, particular performance advantages may reside in the use of non-precious metal-containing stannosilicate catalysts as dehydrogenation catalysts, with tin as a dehydrogenation active metal being incorporated into a base silicate framework. Superior performance in terms of catalyst life may correspond to superior catalyst stability, or resistance to deactivation (*e.g.,* through coking). Catalyst stability may be measured, for example, according to the rate of performance decrease (*e.g.,* conversion decrease, selectivity decrease, and/or yield decrease) over the course of a given test, such as an accelerated stability test, in which constant operating conditions are maintained. Catalyst stability may alternatively be measured according to the rate of operating (*e.g.,* catalyst bed) temperature increase, as needed to maintain a given performance (*e.g.,* conversion, selectivity, and/or yield) over the course of a given test, such as an accelerated stability test. In some cases, superior performance in terms of combined conversion and selectivity may be attained by an increase in selectivity that overcomes (overcompensates for) a decrease in conversion, such that yield is increased. This yield increase, associated with operation at increased selectivity albeit at decreased conversion, may also be accompanied by increased catalyst life or stability, for example due to a reduced rate of formation of coke precursors that lead to catalyst coking and deactivation. Such superior performance (*e.g.,* in terms of conversion, selectivity, yield, and/or catalyst life) may be attained relative to a comparative catalyst system, or bed of such system, that is equivalent in all respects, except that, in the comparative catalyst system, an equivalent amount of a conventional dehydrogenation catalyst (*e.g.,* a catalyst composition of 0.3 wt-% Pt, 0.6 wt-% Sn, on an alumina support) is substituted for the dehydrogenation catalyst. The comparative catalyst system in this case is used in a baseline process, as described herein, that similar in all respects to the process used to obtain the superior performance with catalysts and catalyst systems as described herein.

### Dehydrogenation and Metathesis Processes

Representative dehydrogenation and metathesis processes comprise contacting a feed comprising one or more paraffinic hydrocarbons such as propane with a dehydrogenation and metathesis catalyst or catalyst system as described herein, to convert at least a portion of the paraffinic hydrocarbon and provide a product comprising at least one, and typically at least two, olefinic hydrocarbons having different carbon numbers, relative to the paraffinic hydrocarbon. This "paraffinic hydrocarbon" of the feed, in view of the description of two olefinic hydrocarbons having different carbon numbers, may refer to one particular hydrocarbon, such as propane, that is present in the feed at a predominant concentration as described above, possibly in a mixture with one or more other paraffinic hydrocarbons (*e.g.,* butane) that are present at lower concentrations. In the case of the paraffinic hydrocarbon being propane, for example, two olefinic hydrocarbons present in the product, as a result of dehydrogenation and metathesis, may be ethylene and butene.

In terms of process performance, the values of conversion and selectivity described herein may, according to some embodiments, refer to the conversion of the particular paraffinic hydrocarbon that is present in the feed at the predominant concentration and to the selectivity to one or two olefinic hydrocarbons produced from dehydrogenation and metathesis of that paraffinic hydrocarbon. In other embodiments, the values of conversion and selectivity may refer to the conversion of more than one, or all, paraffinic hydrocarbons present in the feed and/or the selectivity to more than one, or all, olefinic hydrocarbons produced from dehydrogenation and metathesis of these paraffinic hydrocarbons. The values of conversion and selectivity may therefore independently and respectively refer to (i) the conversion of one or more, or all, paraffinic hydrocarbons in the feed and (ii) the selectivity to one or more, or all, olefinic hydrocarbons in the product. The conversion may be calculated, for example, by determining the weight, or weight per unit time in the case of continuous flow processes, of one or more paraffinic hydrocarbons in both the feed and the product (WPar_{feed} and WPar_{prod}), such that the conversion has a value of 1-(WPar_{prod}/WPar_{feed}), expressed as a percentage. The selectivity to one or more olefinic hydrocarbons may be calculated by determining the weight, or weight per unit time, of the one or more olefinic hydrocarbons that have been produced, *i.e.,* weight present in the product that is absent in the feed (WOl_{prod}-WOl_{feed}), and then determining the weight, or weight per unit time, of the total hydrocarbons that have been produced (*i.e.,* excluding the weight of hydrocarbons in the feed that remain unconverted in the product) (WHC_{prod}-WHC_{feed}), such that the selectivity has a value of (WOl_{prod}-WOl_{feed}) / (WHC_{prod}-WHC_{feed}), expressed as a percentage. The yield of one or more olefinic hydrocarbons may be determined as the weight, or weight per unit time, of the one or more olefinic hydrocarbons produced, divided by the weight, or weight per unit time, of the one or more paraffinic hydrocarbons in the feed (*i.e.*, the weight of those paraffinic hydrocarbons that could theoretically be converted to yield the one or more olefinic hydrocarbons), expressed as a percentage. For purposes of illustration using a simplified, hypothetical example, 4,400 grams (100 moles) of propane in a feed is reacted to produce, in a product, (i) 1,260 grams (45 moles) of ethylene, (ii) 2,520 grams (45 moles) of butene, and (iii) 180 grams (90 moles) of hydrogen, with 440 grams (10 moles) of propane being unconverted. Therefore, 3,780 grams (1,260+2,520) of total hydrocarbons are produced. In this case, propane conversion is 90% (1-(440/4,400)), the selectivity to ethylene is 33% ((1,260-0)/(3,780-0)), the selectivity to butene is 67% ((2,520-0)/(3,780-0)), and the selectivity to total olefins (in this case ethylene and butene combined) is 100%. The yield of ethylene is 29% (1,260/4,400), the yield of butene is 57% (2,520/4,400), and the yield of total olefins is 86% (3,780/4,400).

The performance parameters of conversion, selectivity, and/or yield may be determined on a "per-pass" or "once-through" basis, according to the total material introduced to a given dehydrogenation and metathesis catalyst or catalyst system (*e.g.,* comprising one or more catalyst beds in one or more reactors as described herein) and the total material withdrawn from the system. Often, in view of economic considerations and particularly on a larger (*e.g.,* commercial) scale, representative processes may operate by separating and recycling unconverted paraffinic hydrocarbons. In this case, the performance parameters of conversion, selectivity, and/or yield may be determined on an "overall" basis, according to the net material introduced to the catalyst system (and excluding from the total material introduced a recycle portion that is co-introduced with the net material) and the net material withdrawn from the catalyst system (and excluding from the total material withdrawn a recycle portion that is co-withdrawn with the net material and re-introduced to the catalyst system, such as co-introduced with the net material introduced). By recycling unconverted paraffinic hydrocarbons, the overall conversion may considerably exceed the per-pass conversion. For example, as the extent of recycling increases, and approaches the condition of unconverted paraffinic hydrocarbons being recycled to extinction, the overall conversion approaches 100% (regardless of the per-pass conversion), and in this case the overall yield approaches the overall selectivity. In view of the present disclosure, those skilled in the art will appreciate the economic tradeoffs associated with increased product value due to increased yields, offset by increased costs due to increased recycle requirements. Unless stated otherwise, values of conversion, selectivity, and/or yield are given on a per-pass or once-through basis.

As discussed above, aspects of the invention relate to the use of novel dehydrogenation catalysts and metathesis catalysts, as well as novel catalyst systems comprising these catalysts, to provide advantageous conversion and selectivity profiles, and/or improved operational stability. In this regard, the per-pass conversion multiplied by the per-pass selectivity, calculated as described above, provides a per-pass yield, which directly relates to the efficiency and economic attractiveness with which a process can produce one or more olefinic hydrocarbons, such as ethylene. That is, by utilizing catalysts and/or catalyst systems as described herein, and optionally in combination with feeds that comprise one or more sulfur-bearing compounds (*e.g.,* H₂S), significant increases in selectivity may be attained at the expense of only a relatively small decrease in conversion, such that the overall yield of the process may be augmented and its commercial viability greatly strengthened. In representative embodiments, for example, the selectivity to one or more olefinic hydrocarbons (*e.g.,* the at least two olefinic hydrocarbons having different carbon numbers relative to the paraffinic hydrocarbon that is present in the feed at a predominant concentration) may be generally at least about 5% (*e.g.,* from about 5% to about 95% or from about 50% to about 95%), typically at least about 10% (*e.g.,* from about 10% to about 95% or from about 60% to about 95%), and often at least about 15% (*e.g.,* from about 15% to about 90% or from about 70% to about 90%). According to particular embodiments, the selectivity to olefinic hydrocarbons having from 2-4 carbon numbers (*e.g.,* ethylene, propylene, and butene, in the case of a feed comprising propane) may be generally at least about 55% (*e.g.,* from about 55% to about 99%), typically at least about 60% (*e.g.,* from about 60% to about 96%), and often at least about 65% (*e.g.,* from about 65% to about 94%). According to other particular embodiments, the selectivity to propylene (*e.g.,* in the case of a feed comprising propane, such as in a predominant concentration) may be generally at least about 15% (*e.g.,* from about 15% to about 95%, or from about 50% to about 95%), typically at least about 20% (*e.g.,* from about 20% to about 90% or from about 60% to about 90%), and often at least about 25% (*e.g.,* from about 25% to about 85% or from about 65% to about 85%). According to yet other particular embodiments, the selectivity to ethylene (*e.g.,* in the case of a feed comprising propane, such as in a predominant concentration) may be generally at least about 3% (*e.g.,* from about 3% to about 45% or from about 3% to about 30%), typically at least about 4% (*e.g.,* from about 4% to about 35% or from about 4% to about 20%), and often at least about 5% (*e.g.,* from about 5% to about 25% or from about 5% to about 15%). According to still other particular embodiments, the selectivity to butenes (*e.g.,* in the case of a feed comprising propane, such as in a predominant concentration) may be generally at least about 0.1% (*e.g.,* from about 0.1% to about 50% or from about 2% to about 50%), typically at least about 0.3% (*e.g.,* from about 0.3% to about 40% or from about 3% to about 40%), and often at least about 0.5% (*e.g.,* from about 0.5% to about 30% or from about 7% to about 30%). Further aspects of the invention are associated with catalysts and catalyst systems that effectively result in decreased selectivities to undesired, low value byproducts, including the paraffins methane and ethane, as well as hydrocarbon byproducts having 5 or more carbon atoms ("C₅⁺ hydrocarbons"). According to particular embodiments, the selectivity to methane may be generally less than about 20%, typically less than about 15%, and often less than about 10%. According to other particular embodiments, the selectivity to ethane may be generally less than about 25%, typically less than about 20%, and often less than about 10%. According to yet other particular embodiments, the selectivity to C₅⁺ hydrocarbons may be generally less than about 30%, typically less than about 20%, and often less than about 15%, or even less than about 10%, with the extent of byproduct formation depending on the particular feed, and especially the particular distribution of carbon numbers of hydrocarbons in the feed, as well as the possible addition of a sulfur-bearing compound, among other conditions, as one skilled in the art would appreciate from the present specification.

In other representative embodiments, selectivities with respect to any of the hydrocarbons within the ranges given above are achieved at per-pass conversion levels of the paraffinic hydrocarbon(s) (*e.g.,* the paraffinic hydrocarbon, such as propane, that is present in the feed in the predominant concentration) of generally at least about 20% (*e.g.,* from about 20% to about 65%), typically at least about 25% (*e.g.,* from about 25% to about 55%), and often at least about 30% (*e.g.,* from about 30% to about 55%). According to particular embodiments, per-pass conversion levels and/or selectivities within any of the above ranges may be achieved with a feed comprising propane at a predominant concentration (*e.g.,* greater than about 50 vol-%), such as in the case of a feed comprising all or substantially all (*e.g.,* greater than about 95 vol-%) of liquefied petroleum gas (LPG). As noted above, overall conversion levels in a continuous process can be substantially increased over the per-pass conversion if unconverted paraffinic hydrocarbons are separated from the product and recycled to the feed. If essentially all of such unconverted paraffinic hydrocarbons are converted by recycling to achieve substantially 100% overall conversion (minus some inevitable losses), then the value of the overall yield will approach that of the selectivity.

In view of these per-pass conversion and selectivity values, the per-pass yield of the one or more olefinic hydrocarbons (*e.g.,* the per-pass yield of olefinic hydrocarbons having from 2-4 carbon numbers, or otherwise the at least two olefinic hydrocarbons having different carbon numbers relative to the paraffinic hydrocarbon that is present in the feed at a predominant concentration), may be generally at least about 10% (*e.g.,* from about 10% to about 80%), typically at least about 15% (*e.g.,* from about 15% to about 75%), and often at least about 17% (*e.g.,* from about 17% to about 65%). As described above, particular catalysts such as dehydrogenation catalysts comprising a stannosilicate, as well as catalyst systems comprising such catalysts, can provide significant benefits in terms of selectivity and consequently yield, in addition to benefits in terms of stability, *i.e.,* the ability to maintain desired levels of stability and yield over extended periods of operation. For example, in representative embodiments, the selectivity to, or yield of, the one or more olefinic hydrocarbons (*e.g.,* the at least two olefinic hydrocarbons having different carbon numbers relative to the paraffinic hydrocarbon that is present in the feed at a predominant concentration) may be increased in value by a difference in percentage compared to a baseline process utilizing a comparative catalyst system as described above (*i.e.*, the increase represented by the selectivity % of the process utilizing a catalyst or catalyst system as described herein, minus the selectivity % of the baseline process utilizing the comparative catalyst system, or the increase represented by the yield % of the process utilizing a catalyst or catalyst system as described herein minus the yield % of the baseline process utilizing the comparative catalyst system) of generally at least about 3% (*e.g.,* from about 3% to about 35%), typically at least about 5% (*e.g.,* from about 5% to about 30%), and often at least about 7% (*e.g.,* from about 7% to about 15%). For example, the increases in these ranges over a baseline process utilizing the comparative catalyst system may represent increases in selectivity to olefinic hydrocarbons having from 2-4 carbon numbers, or otherwise increases in selectivity to propylene, ethylene, or butenes. In such embodiments, the baseline process is similar in all respects (catalysts, feed composition, reactor configuration, process conditions, *etc*.), with the exception that a comparative catalyst system is utilized.

Contacting between the feed and catalyst system can be performed in a batchwise (discontinuous) manner, but, for the sake of process efficiency, normally a continuous flow of feed is input to the catalyst or catalyst system, such as in the form of one or more catalyst beds as described herein (*e.g.,* as fixed beds or possibly a fluidized bed) and contained within a dehydrogenation/metathesis reactor, and a continuous flow of product is withdrawn from the catalyst or catalyst system. In the case of a catalyst system having two or more fixed beds, any two or more of such beds may be contained within a single dehydrogenation/metathesis reactor, for example divided using a layer of inert material or otherwise stacked directly adjacent one another. In such embodiments, adjacent fixed beds of catalyst may also be referred to as adjacent "layers" of catalyst. Alternatively, any two or more of such catalyst beds may be contained within separate dehydrogenation reactors, dehydrogenation/metathesis reactors, or metathesis reactors, for example if separate reaction conditions (*e.g.,* temperature, pressure, and/or weight hourly space velocity) are desired for such beds. Therefore, various configurations are possible in the case of a catalyst system having three fixed beds, such as first, second and third beds, with the first bed being disposed upstream of the second bed and the third bed being disposed downstream of the second bed, with the relative positional terms "upstream" and "downstream" being with respect to a direction of flow of the feed. For example (i) all three beds may be contained within a single reactor, (ii) the first and second beds may be contained in an upstream reactor and the third bed in a downstream reactor, (iii) the first bed may be contained in an upstream reactor and the second and third beds may be contained in a downstream reactor, or (iv) each of the three beds may be contained in a separate reactor. In view of this description, the possibilities for configurations of catalyst systems having four or more beds, although more numerous, are nonetheless apparent. Overall, it can be appreciated that numerous possibilities exist, with respect to individual beds of a catalyst system, their upstream or downstream position relative to one another, their containment in separate reactors or in combination in a single reactor, and their content of dehydrogenation catalyst and/or metathesis catalyst.

In the case of a continuous flow system, the feed, comprising paraffinic hydrocarbon(s) as described above, can refer to a single feed stream or a total combined feed stream that includes, for example, other sources of paraffinic hydrocarbon(s) such as a recycle stream as described above that comprises an unconverted portion of the paraffinic hydrocarbon(s) that has been separated from the product. The hydrocarbon feed may, but does not necessarily, comprise only paraffinic hydrocarbons. For example, the feed generally comprises predominantly (*i.e.,* at least 50% by weight) paraffinic hydrocarbons, typically comprises at least about 80% (*e.g.,* from about 80% to about 100%) paraffinic hydrocarbons, and often comprises at least about 90% (*e.g.,* from about 90% to about 100% by weight) paraffinic hydrocarbons. Any of these ranges may refer to a combined amount of paraffinic hydrocarbons (*e.g.,* a combined amount of propane and butane) present in the feed, or these ranges may alternatively refer to a particular paraffinic hydrocarbon, such as propane or butane. Light paraffinic hydrocarbons, *e.g.,* C₂-C₆ paraffins, that may be present in a feed as described herein, may be obtained as products or fractions from crude oil refining, such as light gas oil, including liquefied petroleum gas (LPG) and naphtha. Naphtha, including fractions referred to in the art as "light naphtha" and "heavy naphtha," may be more broadly the source of a feed comprising C₂-C₁₂ paraffinic hydrocarbons. For example, light naphtha may be a source of a feed comprising C₂-C₆ paraffinic hydrocarbons or a feed comprising C₅-C₆ paraffinic hydrocarbons, depending on the boiling range fraction of hydrocarbons of interest. Heavy naphtha may be a source of a feed comprising C₆-C₁₂ paraffinic hydrocarbons. Representative feeds may therefore comprise paraffinic hydrocarbons having carbon numbers within any of these ranges. Of the overall weight of hydrocarbons present in the feed, generally at least 50% by weight, such as from about 60% to about 100% by weight, or even from about 90% to 100% by weight, are paraffinic hydrocarbons. In many cases all or a large proportion, such as from about 80% to about 100% or even from about 90% to about 100%, of the paraffinic hydrocarbons in the feed are a paraffinic hydrocarbon having a particular carbon number, such as a carbon number of 3 or 4 (in the case of propane or butane, respectively). Otherwise, such large proportion, in the ranges given above, may apply to a combined amount of paraffinic hydrocarbons having a range of carbon numbers, such as carbon numbers of 2-5 (a combined amount of ethane, propane, butane, and pentane), carbon numbers of 2-4 (a combined amount of ethane, propane, and butane), or carbon numbers of 3-4 (a combined amount of propane and butane). In the case of a feed comprising LPG, for example, the combined amount of propane and butane may represent at least about 90 vol-% or mol-% of all paraffinic hydrocarbons in the feed. The feed may comprise from about 30 vol-% to about 85 vol-%, such as from about 40 vol-% to about 75 vol-%, propane and from about 15 vol-% to about 70 vol-%, such as from about 25 vol-% to about 60 vol-% butane, with the butane content including both normal butane and isobutene. Generally, propane may be present in a predominant concentration (*e.g.,* a concentration greater than about 50 vol-%) in the feed. Preferably, cyclic paraffinic hydrocarbons (*e.g.,* cyclopentane and/or cyclohexane) are present in a minor amount, or minor combined amount, of the feed, for example in an amount, or combined amount, of less than 10% by weight, or less than 1% by weight, of the feed. The feed may comprise an oxidizing agent (*e.g.,* oxygen, air, or a peroxide such as H₂O₂), in the case of the dehydrogenation reaction (such as reaction 1 above) being an oxidative dehydrogenation reaction. Preferably, however, the feed comprises no or substantially no oxidizing agent, or otherwise the dehydrogenation and metathesis process is performed in the absence or substantial absence of any oxidizing agent being introduced to a reactor used in this process. For example, any oxidizing agent (*e.g.,* oxygen, air, or a peroxide such as H₂O₂) may be introduced to the process, or to a reactor used in this process, in an amount (or at a rate) representing less than about 0.5 wt-%, less than about 0.1 wt-%, or even less than 0.01 wt-%, of the feed weight (or feed rate). The term "oxidizing agent" does not encompass impurity levels of oxygenated hydrocarbons (*e.g.,* ethers, aldehydes, or carboxylates) that may be present in the feed (*e.g.,* at less than about 0.1% by weight) and that generally do not participate in oxidation reactions to any appreciable extent.

According to some embodiments, in addition to a paraffinic hydrocarbon as described above, the feed may further comprise one or more sulfur-bearing compounds, such as a mercaptan compound (*i.e*., a thiol, for example methanethiol) or a disulfide compound (for example dimethyl disulfide). A preferred sulfur-bearing compound is H₂S. Through the use of such one or more sulfur-bearing compounds, the concentration of sulfur in the feed may be maintained in an amount effective to provide the performance advantages as described herein. In representative embodiments, the feed has a total sulfur concentration (*e.g.,* due to the presence of H₂S) from about 5 parts per million by volume (vol-ppm) to about 500 vol-ppm, and preferably from about 10 vol-ppm to about 250 vol-ppm. Often, a total sulfur concentration from about 15 vol-ppm to about 150 vol-ppm, such as from about 15 vol-ppm to about 75 vol-ppm, is sufficient to obtain such performance advantages. In the case of H₂S as the sulfur-bearing compound, this may be added to a dehydrogenation and metathesis process *(e.g.,* combined with the feed) as H₂S, or otherwise may be added as an H₂S-precursor, such as an organic sulfide (*e.g.,* dimethyl disulfide, DMDS or carbon disulfide, CS₂), with such H₂S-precursor generating H₂S under conditions of the dehydrogenation and metathesis process. In particular, a suitable H₂S-precursor decomposes at elevated temperatures and in a hydrogen atmosphere, to form H₂S. For example, DMDS decomposes to form H₂S and CH₄, according to the reaction:

CH₃S₂CH₃ + 3H₂ → 2 CH₄ + 2 H₂S.

Suitable H₂S precursors are preferably organic sulfur-containing liquids, such as DMDS, that facilitate handling of process sulfur requirements, compared to the handling of hazardous H₂S in gas form.

As described above, the addition of sulfur-bearing compounds, particularly in view of characteristics of catalysts and catalyst systems described herein (*e.g.,* substantially lacking any precious metals such as Pt), can provide performance advantages, especially in terms of higher selectivity and yield of C₂-C₄ olefins and/or lower selectivity and yield of methane and/or ethane. Such performance advantages may be determined relative to baseline process is similar in all respects, including the use of the same catalyst or catalyst system described herein, with the exception of the sulfur-bearing compound being absent from the feed in the baseline process. Accordingly, performance advantages may be characterized as being obtained relative to a reference feed that is the same in all respects but that lacks the sulfur-bearing compound, in a baseline process that is the same in all respects, including conditions (temperature, pressure, weight hourly space velocity). For example, in representative embodiments, the selectivity to, or yield of, the one or more olefinic hydrocarbons (*e.g.,* the at least two olefinic hydrocarbons having different carbon numbers relative to the paraffinic hydrocarbon that is present in the feed at a predominant concentration) may be increased in value by a difference in percentage compared to a reference feed in a baseline process (*i.e.,* the increase represented by the selectivity % obtained with the sulfur-containing feed minus the selectivity % obtained with the reference feed in the baseline process or the increase represented by the yield % obtained with the sulfur-containing feed minus the yield % obtained with the reference feed in the baseline process) of generally at least about 1% (*e.g.,* from about 1% to about 10%), and typically at least about 3% (*e.g.,* from about 3% to about 8%), due to the presence of sulfur in the feed. In other representative embodiments, the selectivity to, or yield of, methane and/or ethane may be decreased in value by a difference in percentage compared to a reference feed in a baseline process (*i.e*., the decrease represented by the selectivity % obtained with the reference feed in the baseline process minus the selectivity % obtained with the sulfur-containing feed or the decrease represented by the yield % obtained with the reference feed in the baseline process minus the yield % obtained with the reference feed) of generally at least about 1% (e.g., from about 1% to about 10%), and typically at least about 3% (*e.g.,* from about 3% to about 8%), due to the presence of sulfur in the feed.

Representative dehydrogenation and metathesis processes, for example to achieve the conversion, selectivity, and/or yield values as described herein, therefore comprise contacting a feed, either continuously or batchwise, with a catalyst or catalyst system as described herein. Generally, the contacting is performed with the feed being passed continuously through a catalyst or catalyst system as described above, and preferably having at least one (and possibly only one) fixed bed of a mixture of the catalysts, and preferably a uniform mixture, in a reactor or reaction zone, normally under conditions effective for achieving the desired reaction sequence. In some cases, a swing bed system may be utilized, in which the flowing paraffinic hydrocarbon-containing feed is periodically re-routed to (i) bypass one or more beds of catalyst that have become spent or deactivated (spent catalyst system) and (ii) contact one or more beds of fresh catalyst (fresh catalyst system). A number of other suitable configurations for carrying out the feed/catalyst contacting are known in the art, with the optimal choice depending on the particular feed, rate of catalyst deactivation, and other factors. Such configurations include moving bed configurations (*e.g.,* counter-current flow configurations, radial flow configurations, *etc.*) and fluidized bed configurations, any of which may be integrated with batchwise or continuous catalyst regeneration, as is known in the art. Regeneration refers to the removal, by combustion that is performed generally in an oxygen environment within a separate regenerator vessel, of accumulated deposits of carbon (coke) from spent catalyst, in order to provide fresh or regenerated catalyst for additional processing of feed.

Representative conditions for contacting of the feed with the catalyst or catalyst system, at which the above per-pass conversion, selectivity, and yield may be obtained, include a temperature generally from about 350°C to about 800°C, typically from about 500°C to about 650°C, and often from about 550°C to about 625°C; an absolute pressure generally from about 0.1 bar to about 100 bar, typically from about 1 bar to about 50 bar, and often from about 1 bar to about 25 bar; and a weight hourly space velocity (WHSV) generally from about 0.01 hr⁻¹ to about 40 hr⁻¹, typically from about 0.01 hr⁻¹ to about 30 hr⁻¹, and often from about 0.1 hr⁻¹ to about 25 hr⁻¹. As is understood in the art, the WHSV is the weight flow of the feed divided by the weight of the catalyst (*e.g.,* total weight of catalyst in the catalyst system, which catalyst is present in one or more beds as described above) and represents the equivalent catalyst weights of feed processed every hour. The WHSV is related to the inverse of the reactor residence time. Under the reaction conditions, the feed is normally partially or all in the vapor phase in the reactor or reaction zone containing the catalyst or catalyst system, but it may also be in the liquid phase, depending on the particular process conditions and feed used.

Prior to the contacting with the feed, the catalyst or catalyst system may be pretreated, by contacting it with an oxidizing agent and/or reducing agent (*e.g.,* an oxidizing gas or reducing gas), in order to convert or obtain the dehydrogenation active metal(s) and/or olefin metathesis active metal(s) in a desired form (*e.g.,* an oxidized form or a reduced form) for use in the dehydrogenation and metathesis process. According to a particular embodiment, the catalyst or catalyst system is pretreated by contacting it with an oxidizing agent such as oxygen or an oxygen-containing gas (*e.g.,* air), followed by contacting with a reducing agent, such as hydrogen or a hydrogen-containing gas (*e.g.,* a mixture of hydrogen and nitrogen). Preferably contacting step(s) used in pretreating is performed with flowing streams of the oxidizing agent and/or reducing agent.

The following examples are set forth as representative of the present invention. These examples are not to be construed as limiting the scope of the invention as other equivalent embodiments will be apparent in view of the present disclosure and appended claims.

### EXAMPLES

Experiments were conducted to evaluate catalyst systems for performing (i) the dehydrogenation of propane to propylene and (ii) the metathesis of propylene to ethylene and butene. This integration of reactions was also termed "propane transformation." The catalyst systems tested included those having both a dehydrogenation catalyst and a metathesis catalyst. In many examples, a catalyst bed was utilized in which the dehydrogenation catalyst and the metathesis catalyst were mixed uniformly. Some examples also utilized a downstream catalyst bed of the metathesis catalyst only, and other examples also utilized both an upstream catalyst bed of the dehydrogenation catalyst only and a downstream catalyst bed of the metathesis catalyst only. In the examples termed "comparative," a platinum-containing dehydrogenation catalyst was used, whereas in the examples termed "inventive," a mesoporous stannosilicate catalyst was used. The designation of an example as "Comparative" or "Reference" is not meant to imply that the catalyst systems used in the comparative examples or the catalysts used in the reference examples are conventional or known. In fact, the catalyst systems tested in the comparative examples are within certain embodiments of the present invention. The term "Comparative" in the case of these examples is merely to indicate their use as a comparative indicator of for performance testing purposes, and in particular for a comparison of the performance of non-precious metal catalysts against the performance of precious metal catalysts. For the catalyst systems evaluated, the designations used for the dehydrogenation catalysts and metathesis catalysts and their corresponding compositions are summarized in Table 1.

**Table 1-Catalyst Designations**

| **Catalyst Type** | **Designation** | **Description / Composition** |
|---|---|---|
| dehydrogenation | DEHY-COMP A | 0.3 wt-% Pt / 0.6 wt-% Sn, supported on gamma Al₂O₃, prepared by metal impregnation onto the support |
| dehydrogenation | DEHY-COMP B | 0.3 wt-% Pt / 0.6 wt-% Sn, supported on mixed MgO-Al₂O₃ that was obtained from calcination of an LDH, prepared by metal impregnation onto the support |
| dehydrogenation | DEHY-COMP C | 0.3 wt-% Pt / 0.6 wt-% Sn, supported on SiO₂, prepared by metal impregnation onto the support |
| dehydrogenation | DEHY-COMP D | 0.3 wt-% Pt, supported on SiO₂, prepared by metal impregnation onto the support |
| dehydrogenation | DEHY-INV A | mesoporous stannosilicate, having 0.5 wt-% tin incorporated into a base silicate framework, prepared by precipitation |
| dehydrogenation | DEHY-INV B | mesoporous stannosilicate, having 1 wt-% tin incorporated into a base silicate framework, prepared by precipitation |
| dehydrogenation | DEHY-INV C | mesoporous stannosilicate, having 5 wt-% tin incorporated into a base silicate framework, prepared by precipitation |
| dehydrogenation | DEHY-INV D | mesoporous stannosilicate, having 10 wt-% tin incorporated into a base silicate framework, prepared by precipitation |
| dehydrogenation | DEHY-INV E | 1 wt-% In / 10 wt-% Sn, supported on SiO₂, prepared by metal impregnation onto the support |
| dehydrogenation | DEHY-INV F | 1 wt-% Ga / 10 wt-% Sn, supported on SiO₂, prepared by metal impregnation onto the support |
| dehydrogenation | DEHY-INV G | 10 wt-% Sn, supported on SiO₂, prepared by metal impregnation onto the support |
| metathesis | META A | 8 wt-% tungsten, as WO₃, supported on SiO₂ |
| metathesis | META B | 8 wt-% tungsten, as WO₃, supported on 5 wt-% hydrogen form zeolite Y (HY zeolite), 10 wt-% mixed MgO-Al₂O₃ obtained from calcination of an LDH, balance SiO₂ |
| metathesis | META C | 8 wt-% tungsten, as WO₃, supported on a mixture of 5 wt-% hydrogen form zeolite Y (HY zeolite), with the balance of the catalyst weight being SiO₂ |

### Preparation of Dehydrogenation Catalysts and Metathesis Catalysts

The catalyst designated as DEHY-COMP A was prepared as follows: A solution of chloroplatinic acid and tin (II) chloride dihydrate was loaded into particles of a gamma alumina support, using an incipient wetness impregnation method. The material obtained was then dried at 120°C (248°F) for 12 hours and calcined under an air environment at 620°C (1148°F) for 2 hours. The resulting catalyst contained 0.3 wt-% Pt and 0.6 wt-% Sn, on the gamma-alumina support, with these weight percentages being based on the total catalyst weight. The catalyst designated DEHY-COMP B was prepared in a similar manner, except that the support used was obtained by calcination of Mg-Al-CO₃ layered double hydroxide under an air environment at 620°C (1148°F) for 8 hours. The catalysts designated DEHY-COMP C, DEHY-COMP D, DEHY-INV E, DEHY-INV F, and DEHY-INV G were prepared in a similar manner, except that a silica support was impregnated using the incipient wetness method, with solutions of metals as needed to obtain the metal percentages and metal types according to the descriptions/compositions of these catalysts as shown above in Table 1.

The catalysts designated as DEHY-INV A, DEHY-INV B, DEHY-INV C, and DEHY-INV D were prepared using tetraethylorthosilicate (TEOS) as a source of mesoporous silica (mSiO₂), cetyltrimethylammonium bromide (CTAB) as a templating surfactant, tin(II) chloride dihydrate (SnCl₂·2H₂O) as a source of tin, and an ethanol-water solution as a solvent. Specifically, CTAB was dissolved in the ethanol-water solvent by combining these components and stirring until the mixture became a homogeneous solution. Aqueous ammonium hydroxide was then added to this solution to adjust its pH value to within 8-12, followed by a slow addition of TEOS and SnCl₂·2H₂O. The Sn loading of the catalysts was controlled in the different catalyst preparations at 0.5 wt-% (DEHY-INV A), 1 wt-% (DEHY-INV B), 5 wt-% (DEHY-INV C), and 10 wt-% (DEHY-INV D), by adjusting the amounts of TEOS and SnCl₂·2H₂O. Following the complete addition of the silica and tin sources, the reaction was continued for a period of 3-24 hours. The resulting, precipitated solids were filtered, washed several times with deionized water, and dried at room temperature initially and at 120°C (248°F) for 12 hours subsequently. Finally, the mesoporous stannosilicate catalysts, having at least a portion of the tin being chemically bonded with the silicate base material, were obtained by calcining at 550°C (1022°F) for 6 hours.

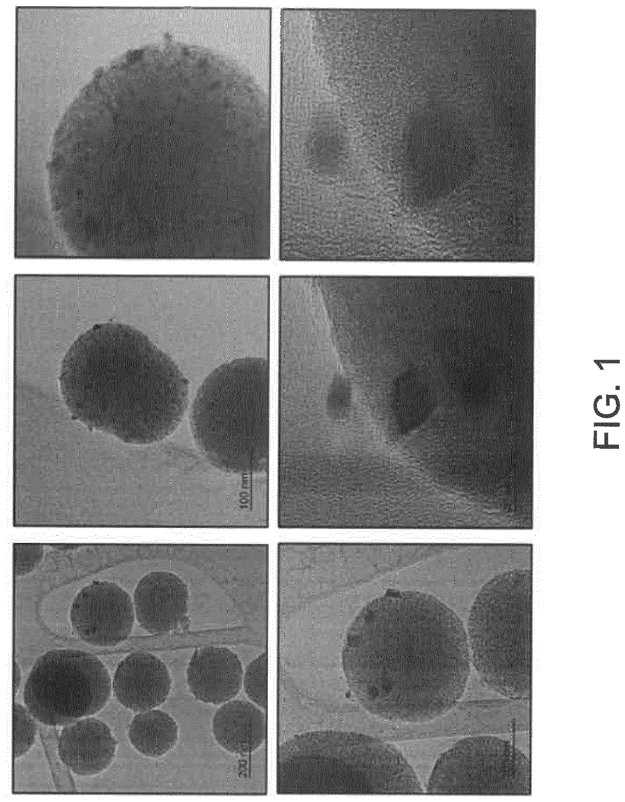

FIG. 1 depicts the transmission electron microscopy (TEM) images of the Sn-containing mesoporous silica, or mesoporous stannosilicate, having 5 wt-% Sn (DEHY-INV C) and synthesized according to this precipitation method. Mesoporosity of the SiO₂ is clearly evident from these images, with certain locations of this mesoporous silica having small SnO₂ nanoparticles, with a size varying from 5-20 nm, being deposited external to the mesopores, including on the surface of the mesoporous silica and otherwise throughout this base material. Observed surface nanoparticles were believed to have resulted from an amount of Sn precursor (SnCl₂·2H₂O) that did not become chemically incorporated into the mesopores of the base material, by forming Sn-O-Si bonds. A high dispersion or distribution of Sn atoms throughout the interior of the mesoporous silica particles, was confirmed by high-angle annular dark-field scanning TEM (HAADF STEM) and atomic-resolved energy dispersive X-ray spectroscopic (EDS) mapping. This was particularly evident from overlaying images of the Sn and Si atoms present in these particles. Likewise, EDS mapping performed on a sample of the mesoporous stannosilicate having 10 wt-% Sn (DEHY-INV D) and also synthesized according to the above precipitation method similarly revealed highly dispersed Sn atoms within the mesoporous silica, as well as deposited SnO₂ nanoparticles, at locations external to the mesopores and including the mesoporous silica surface.

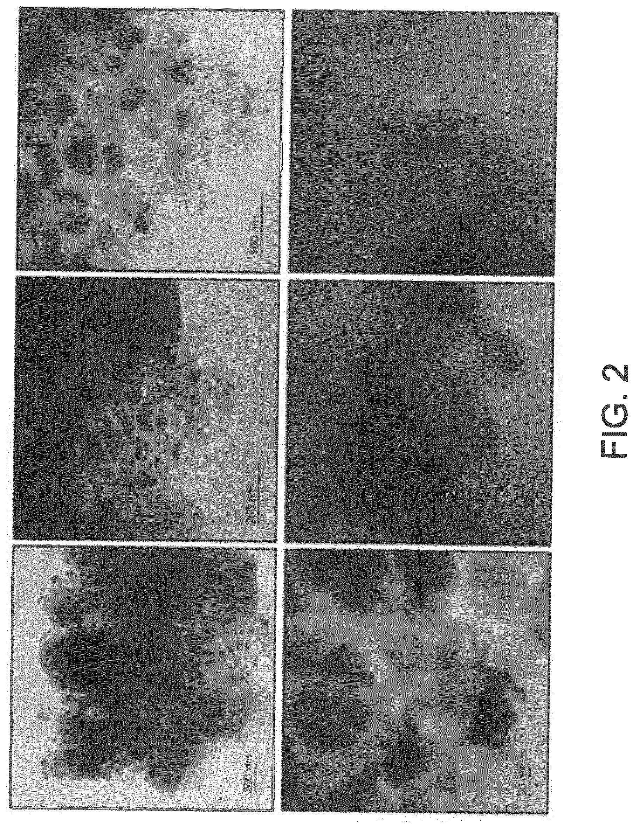

For comparative purposes, FIG. 2 depicts the transmission electron microscopy (TEM) images of the Sn-containing silica, or stannosilicate, having 10 wt-% Sn (DEHY-INV G) and synthesized according to an incipient wetness impregnation method. In contrast to the precipitation method used for forming mesoporous stannosilicates, which resulted in a substantial portion of the Sn being highly dispersed from chemical bonding to the base material through Sn-O-Si bonds, tin impregnation resulted in a dispersion of Sn, primarily as SnO₂ nanoparticles and still having favorable particle sizes within the range of 5-100 nm. HAADF STEM and EDS mapping confirmed a high dispersion of these nanoparticles, despite no substantial chemical incorporation into the base silicate. Even with this lack of Sn-O-Si bond formation, stannosilicates obtained by impregnation nonetheless exhibited high dehydrogenation catalytic activity, particularly with respect to comparable catalysts based on platinum or tin-promoted platinum (namely the catalysts designated as DEHY COMP A, DEHY COMP B, DEHY COMP C, DEHY COMP D).

The catalyst designated as MET A was prepared as follows: A support of SiO₂ was impregnated using an impregnation solution of ammonium metatungstate monohydrate. The impregnated support was then dried at 120°C (248°F) for 12 hours and calcined under air at 550°C (1022°F) for 8 hours to obtain the catalyst, which contained, based on the total catalyst weight, 8 wt-% W, with the balance being SiO₂.

The catalyst designated as META B was prepared as follows: A support was prepared by mixing SiO₂ with hydrogen form of zeolite Y (HY-Zeolite), and particles of this mixture of solids were impregnated using an impregnation solution of ammonium metatungstate hydrate. Following the combining of these particles and impregnation solution, the wet, impregnated particles obtained were dried at 120°C (248°F) for 12 hours and calcined under air at 550°C (1022°F) for 8 hours. In addition, an Mg-Al-CO₃ LDH was calcined under air at 620°C (1148°F) for 8 hours to convert the LDH to an MgO-Al₂O₃ mixed metal oxide (calcined LDH). The calcined, tungsten-impregnated material and MgO-Al₂O₃ mixed metal oxide material were combined to obtain a metathesis catalyst, which contained, based on the total catalyst weight, 8 wt-% W, 5 wt-% HY-zeolite, and 10 wt-% mixed magnesium oxide (MgO)-aluminum oxide (Al₂O₃), with the balance being SiO₂. The catalyst designated META C, was prepared in a similar manner, except that the step of mixing with the Mg-Al-CO₃ layered double hydroxide was omitted and the proportions of the remaining ingredients adjusted, such that the resulting metathesis catalyst contained, based on the total catalyst weight, 8 wt-% W, and 5 wt-% HY-zeolite, with the balance being SiO₂.

### Catalyst Systems Using the Prepared Catalysts

Catalyst systems were evaluated, with most systems including a physical mixture of (A) either (i) a dehydrogenation catalyst that was based on Pt (DEHY-COMP A, DEHY-COMP B, DEHY-COMP C, or DEHY-COMP D, as described in Table 1) and was part of a comparative catalyst system, or (ii) a dehydrogenation catalyst that was based on a mesoporous stannosilicate (DEHY-INV A, DEHY-INV B, DEHY-INV C, or DEHY-INV D as described in Table 1) or Sn-impregnated silica (DEHY-INV E, DEHY-INV F, or DEHY-INV G, as described in Table 1) and was part of an inventive catalyst system, and (B) a metathesis catalyst (META A, META B, or META C as described in Table 1). Some of the catalyst systems also included a bed of only a metathesis catalyst, downstream of the bed of the physical mixture of dehydrogenation and metathesis catalysts. The catalyst systems tested, according to various examples, are summarized in Table 2 below, with the weight ratios of catalysts used in the physical mixture given in relative weights of [dehydrogenation catalyst] : [metathesis catalyst].

**Table 2-Catalyst Systems Evaluated**

| **Example** | **BED-PM Physical Mixture** | **BED-M Metathesis Catalyst** | **Bed Weight Ratio [PM]:[M]** |
|---|---|---|---|
| Inventive Example 1A | DEHY-INV C / META A at 0.375:1 w/w | none | |
| Comparative Example 1A | DEHY-COMP A / META A at 0.375:1 w/w | none | |
| Comparative Example 1B | DEHY-COMP B / META A at 0.375:1 w/w | none | |
| Inventive Example 2A | DEHY-INV C / META B at 0.375:1 w/w | none | |
| Inventive Example 2B | DEHY-INV C / META B at 0.75:1 w/w | META B | 1.75:1 |
| Comparative Example 2A | DEHY-COMP A / META B at 0.375:1 w/w | none | |
| Comparative Example 2B | DEHY-COMP B / META B at 0.375:1 w/w | none | |
| Inventive Example 3A | DEHY-INV D / META C at 0.375:1 w/w | none | |
| Inventive Example 3B | DEHY-INV D / META C at 0.75:1 w/w | META C | 1.75:1 |
| Inventive Example 3C | DEHY-INV B / META C at 0.3 75:1 w/w | none | |
| Inventive Example 3D | DEHY-INV A / META C at 0.375:1 w/w | none | |
| Comparative Example 3A | DEHY-COMP D / META A at 0.375:1 w/w | none | |
| Comparative Example 3B | DEHY-COMP C / META A at 0.375:1 w/w | none | |
| Comparative Example 3C | DEHY-COMP C / META C at 0.375:1 w/w | none | |
| Inventive Example 4A | DEHY-INV E / META C at 0.375:1 w/w | none | |
| Inventive Example 4B | DEHY-INV F / META C at 0.375:1 w/w | none | |
| Inventive Example 4C | DEHY-INV G / META C at 0.375:1 w/w | none | |

For reference purposes, dehydrogenation catalysts in isolation were also tested. Specifically, Reference Example 5A tested DEHY-COMP E in isolation and Reference Example 5B tested DEHY-COMP F in isolation.

### Testing of the Catalyst Systems for Integrated Propane Dehydrogenation/Metathesis

For testing of the catalyst systems described above in Table 2, each system was initially pretreated by heating to 580°C (1076°F) and maintaining this temperature under flowing O₂ for 30 minutes and then under flowing H₂ for 30 minutes. Following this pretreating, each catalyst system was heated to an operating temperature of 600°C (1076°F), and propane flow was then initiated to obtain a WHSV of about 0.31 hr⁻¹ in each case, based on the total weight of catalyst in the catalyst system (or 1.13 hr⁻¹ in each case, based on the weight of the dehydrogenation catalyst only), with an operating pressure of 0.2 bar gauge (about 1.2 bar absolute). Consistent with the catalyst systems as shown above in Table 2, the flowing propane feed was first contacted with the upstream bed of physically mixed, dehydrogenation and metathesis catalysts, and the effluent from this bed then contacted with a downstream bed of only metathesis catalyst, for cases in which such downstream bed was used (Inventive Examples 2B and 3B). As noted above, two control examples (Control Example 5A and Control Example 5B) were performed with dehydrogenation catalyst in isolation, *i.e.,* without any metathesis catalyst.

In the evaluation of each catalyst system, samples of the effluent from that system (*i.e*., exiting the bed of the physical mixture of catalysts, or otherwise exiting the downstream bed of the catalyst designated META B in Inventive Example 2B or exiting the downstream bed of the catalyst designated META C in Inventive Example 3B) were obtained after 1 hour on stream and 8 hours on stream. In the experiments performed using the bed configurations corresponding to Inventive Example 1 (InvEx1A), Comparative Example 1A (CompEx1A), Comparative Example 1B (CompExlB), Inventive Example 2A (InvEx2A), Inventive Example 2B (InvEx2B), Comparative Example 2A (CompEx2A), and Comparative Example 2B (CompEx2B), the catalysts were regenerated and tested under the same testing conditions as described above, with samples obtained again at 1 hour and 8 hours on stream to evaluate a second cycle of testing. Similarly, a third cycle of testing was performed using these bed configurations, following a second regeneration of the catalysts. In each experiment, including the second and third cycle testing experiments, the samples were quantitatively analyzed for their composition in order to determine propane conversion and selectivity to all olefins (including olefins having 5 carbon atoms or greater, C₅⁺), as well as the selectivities to the individual compounds, propylene and ethylene, and selectivity to butene isomers (C₄ olefins). The sample analyses were performed by a gas chromatography instrument, equipped with a flame ionization detector (GC-FID).

The yield of all olefins, as well as the yields of propylene, ethylene, and butene isomers, can be determined as the product of the propane conversion and the respective selectivities in each case (*e.g.,* the yield of all olefins as the product of the propane conversion and selectivity to all olefins, the yield of propylene as the product of propane conversion and selectivity to propylene, the yield of ethylene as the product of propane conversion and selectivity to ethylene, or the yield of butene isomers as the product of propane conversion and selectivity to butene isomers). The following conversion and selectivities to olefin products, as determined from the experimental results, including those of the second and third cycle testing experiments described above, are provided below in Table 3.

**Table 3-Conversion / Olefin Product Selectivities**

| **Example** | **% Propane Conversion** | | **% Selectivity, Total Olefins** | | **% Selectivity, Propylene** | | **% Selectivity, Ethylene** | | **% Selectivity, Butenes** | |
|---|---|---|---|---|---|---|---|---|---|---|
| | 1hr | 8hr | 1hr | 8hr | 1hr | 8hr | 1hr | 8hr | 1hr | 8hr |
| InvEx1A 1^{st} Cycle | 22.5 | 23.6 | 87.9 | 85.4 | 65.5 | 56.3 | 11.7 | 14.1 | 5.5 | 11.6 |
| InvEx1A 2^{nd} Cycle | 21.4 | 23.3 | 82.0 | 84.4 | 53.4 | 53.1 | 15.8 | 14.8 | 11.5 | 12.5 |
| InvEx1A 3^{rd} Cycle | 22.1 | 23.2 | 85.0 | 83.3 | 55.2 | 49.0 | 14.6 | 15.9 | 7.2 | 9.4 |
| CompEx1A 1^{st} Cycle | 42.1 | 24.4 | 43.0 | 63.0 | 24.4 | 28.0 | 4.7 | 16.5 | 6.2 | 6.8 |
| CompEx1A 2^{nd} Cycle | 27.0 | 23.0 | 56.0 | 64.8 | 28.9 | 28.7 | 8.3 | 18.4 | 6.7 | 6.7 |
| CompEx1A 3^{rd} Cycle | 30.0 | 22.4 | 45.4 | 64.6 | 31.8 | 28.8 | 6.3 | 19.4 | 4.7 | 6.8 |
| CompEx1B 1^{st} Cycle | 55.8 | 38.0 | 54.7 | 63.2 | 36.6 | 31.9 | 3.4 | 11.3 | 11.4 | 12.3 |
| CompEx1B 2^{nd} Cycle | 57.5 | 32.5 | 51.5 | 64.8 | 26.6 | 32.5 | 4.4 | 13.3 | 11.0 | 10.8 |
| CompEx1B 3^{rd} Cycle | 58.5 | 30.6 | 60.6 | 75.6 | 37.8 | 40.1 | 3.8 | 14.2 | 13.3 | 12.6 |
| InvEx2A 1^{st} Cycle | 30.1 | 34.0 | 74.6 | 71.5 | 49.6 | 37.1 | 14.8 | 16.6 | 7.6 | 11.8 |
| InvEx2A 2^{nd} Cycle | 30.9 | 35.6 | 65.0 | 68.7 | 34.7 | 34.7 | 16.1 | 16.7 | 10.5 | 11.8 |
| InvEx2A 3^{rd} Cycle | 34.5 | 36.0 | 69.1 | 68.1 | 34.6 | 32.9 | 16.8 | 16.5 | 8.8 | 8.6 |
| InvEx2B 1^{st} Cycle | 35.6 | 37.6 | 70.7 | 72.3 | 36.8 | 34.7 | 17.2 | 18.0 | 12.3 | 7.5 |
| InvEx2B 2^{nd} Cycle | 35.6 | 39.1 | 69.1 | 67.8 | 32.2 | 30.4 | 18.0 | 17.7 | 12.6 | 11.8 |
| InvEx2B 3^{rd} Cycle | 34.8 | 38.9 | 67.3 | 66.6 | 31.7 | 29.1 | 18.2 | 17.5 | 12.1 | 8.6 |
| CompEx2A 1^{st} Cycle | 47.8 | 27.7 | 38.8 | 58.0 | 22.6 | 24.8 | 2.2 | 15.6 | 4.9 | 6.5 |
| CompEx2A 2^{nd} Cycle | 34.8 | 25.8 | 51.3 | 61.8 | 29.8 | 26.0 | 6.2 | 18.7 | 7.0 | 6.7 |
| CompEx2A 3^{rd} Cycle | 26.1 | 26.1 | 57.2 | 61.8 | 31.0 | 25.8 | 12.1 | 19.8 | 6.6 | 6.6 |
| CompEx2B 1^{st} Cycle | 50.6 | 40.2 | 54.0 | 61.8 | 40.9 | 31.0 | 1.6 | 11.6 | 4.3 | 12.1 |
| CompEx2B 2^{nd} Cycle | 59.2 | 36.0 | 48.4 | 62.4 | 28.4 | 32.5 | 3.0 | 11.7 | 9.1 | 10.8 |
| CompEx2B 3^{rd} Cycle | 59.5 | 33.2 | 46.1 | 64.2 | 27.6 | 32.2 | 2.3 | 13.6 | 10.7 | 10.3 |
| Inventive Example 3A | 34.4 | 39.9 | 79.6 | 80.0 | 55.8 | 47.6 | 9.9 | 11.5 | 10.2 | 16.0 |
| Inventive Example 3B | 33.8 | 38.3 | 84.9 | 80.5 | 59.0 | 39.4 | 10.7 | 15.1 | 11.4 | 17.6 |
| Inventive Example 3C | 13.9 | 16.4 | 71.9 | 75.5 | 36.9 | 33.4 | 25.1 | 25.3 | 7.5 | 10.5 |
| Inventive Example 3D | 12.2 | 13.8 | 77.4 | 78.5 | 38.9 | 36.2 | 27.3 | 26.7 | 7.9 | 9.6 |
| Comparative Example 3A | 10.0 | 10.4 | 78.0 | 79.7 | 38.4 | 41.4 | 29.1 | 29.3 | 7.2 | 5.4 |
| Comparative Example 3B | 14.8 | 13.9 | 85.0 | 85.4 | 51.2 | 52.4 | 21.7 | 22.4 | 9.0 | 7.6 |
| Comparative Example 3C | 14.0 | 14.3 | 78.5 | 81.1 | 48.3 | 47.6 | 22.2 | 24.1 | 5.3 | 6.8 |
| Inventive Example 4A | 24.5 | 27.6 | 85.0 | 85.0 | 68.6 | 73.3 | 9.5 | 7.7 | 4.9 | 2.5 |
| Inventive Example 4B | 23.4 | 28.7 | 79.8 | 82.8 | 59.8 | 58.6 | 11.8 | 11.2 | 6.0 | 9.3 |
| Inventive Example 4C | 24.2 | 29.9 | 85.7 | 84.2 | 67.8 | 53.9 | 11.1 | 12.3 | 3.6 | 11.4 |
| Control Example 5A | 22.3 | 20.8 | 93.5 | 92.9 | 85.4 | 82.8 | 6.5 | 7.6 | 0.5 | 0.5 |
| Control Example 5B | 21.7 | 20.8 | 92.8 | 91.8 | 85.4 | 82.7 | 6.5 | 7.7 | 0.3 | 0.3 |

Also determined from the sample analyses, but not shown in Table 3, were the selectivities to undesired, low value byproducts, including paraffins. The selectivity to all paraffins, as well as the selectivities to the individual paraffins, methane and ethane, which result from cracking and/or hydrogenation side reactions, are provided below in Table 4. Also provided in this Table are the selectivities to hydrocarbon byproducts having 5 or more carbon atoms, *i.e.,* C₅⁺ hydrocarbon byproducts ("C₅⁺ HCBNS").

**Table 4-Paraffin / C₅⁺ Hydrocarbon Selectivities**

| **Example** | **% Selectivity Total Paraffins** | | **% Selectivity, Ethane** | | **% Selectivity, Methane** | | **% Selectivity, C₅⁺ HCBNS** | |
|---|---|---|---|---|---|---|---|---|
| | 1hr | 8hr | 1hr | 8hr | 1hr | 8hr | 1hr | 8hr |
| InvEx1A 1^{st} Cycle | 12.1 | 14.6 | 5.7 | 7.6 | 6.0 | 6.5 | 5.2 | 3.4 |
| InvEx1A 2^{nd} Cycle | 18.0 | 15.6 | 10.0 | 8.3 | 7.2 | 6.8 | 1.3 | 3.9 |
| InvEx1A 3^{rd} Cycle | 15.0 | 16.7 | 7.7 | 8.9 | 6.8 | 7.1 | 6.7 | 8.9 |
| CompEx1A 1^{st} Cycle | 57.0 | 37.0 | 31.1 | 15.4 | 22.8 | 24.4 | 7.8 | 11.8 |
| CompEx1A 2^{nd} Cycle | 44.0 | 35.2 | 21.6 | 14.2 | 20.5 | 19.7 | 12.0 | 10.9 |
| CompEx1A 3^{rd} Cycle | 54.6 | 35.4 | 26.8 | 13.9 | 25.2 | 20.2 | 2.6 | 9.6 |
| CompEx1B 1^{st} Cycle | 45.3 | 36.8 | 30.7 | 23.3 | 9.0 | 11.1 | 3.2 | 7.5 |
| CompEx1B 2^{nd} Cycle | 48.5 | 35.2 | 34.5 | 20.9 | 8.6 | 12.6 | 9.4 | 8.2 |
| CompEx1B 3^{rd} Cycle | 39.4 | 24.4 | 28.5 | 15.2 | 5.6 | 7.7 | 5.5 | 8.9 |
| InvEx2A 1^{st} Cycle | 25.4 | 28.5 | 11.7 | 15.3 | 13.0 | 12.3 | 2.6 | 5.9 |
| InvEx2A 2^{nd} Cycle | 35.0 | 31.3 | 18.8 | 17.3 | 14.8 | 13.1 | 3.6 | 5.3 |
| InvEx2A 3^{rd} Cycle | 30.9 | 31.9 | 16.7 | 17.7 | 13.2 | 13.2 | 8.8 | 10.2 |
| InvEx2B 1^{st} Cycle | 29.3 | 27.6 | 14.7 | 14.8 | 12.9 | 11.8 | 4.1 | 8.8 |
| InvEx2B 2^{nd} Cycle | 31.9 | 32.2 | 17.3 | 18.5 | 13.1 | 12.6 | 5.3 | 7.8 |
| InvEx2B 3^{rd} Cycle | 32.7 | 33.4 | 17.5 | 19.3 | 13.6 | 12.9 | 5.1 | 11.3 |
| CompEx2A 1^{st} Cycle | 61.2 | 42.0 | 34.3 | 18.3 | 23.4 | 22.6 | 8.9 | 11.0 |
| CompEx2A 2^{nd} Cycle | 48.7 | 38.2 | 25.7 | 15.2 | 20.9 | 21.9 | 8.3 | 10.4 |
| CompEx2A 3^{rd} Cycle | 42.8 | 38.2 | 19.5 | 14.6 | 22.1 | 22.5 | 7.6 | 9.5 |
| CompEx2B 1^{st} Cycle | 46.0 | 38.2 | 29.6 | 23.8 | 12.6 | 12.7 | 7.1 | 7.0 |
| CompEx2B 2^{nd} Cycle | 51.6 | 37.6 | 36.4 | 22.4 | 9.7 | 13.8 | 7.6 | 7.2 |
| CompEx2B 3^{rd} Cycle | 53.9 | 35.8 | 37.7 | 20.0 | 10.1 | 14.6 | 5.2 | 7.9 |
| Inventive Example 3A | 20.4 | 20.0 | 10.9 | 12.2 | 7.9 | 6.4 | 3.6 | 5.8 |
| Inventive Example 3B | 15.1 | 19.5 | 8.0 | 12.7 | 6.2 | 6.5 | 3.8 | 8.4 |
| Inventive Example 3C | 28.1 | 24.5 | 8.0 | 8.2 | 18.5 | 17.0 | 2.2 | 6.4 |
| Inventive Example 3D | 22.6 | 21.5 | 5.5 | 6.1 | 16.3 | 14.9 | 3.3 | 5.9 |
| Comparative Example 3A | 22.0 | 20.3 | 4.8 | 4.0 | 16.9 | 16.0 | 3.3 | 3.6 |
| Comparative Example 3B | 15.0 | 14.6 | 4.5 | 3.8 | 10.2 | 13.9 | 3.2 | 3.1 |
| Comparative Example 3C | 21.5 | 18.9 | 6.5 | 5.9 | 14.4 | 14.1 | 2.6 | 4.3 |
| Inventive Example 4A | 15.0 | 15.0 | 7.2 | 8.3 | 7.4 | 7.2 | 2.0 | 1.9 |
| Inventive Example 4B | 20.2 | 17.2 | 10.4 | 10.9 | 9.0 | 8.1 | 2.1 | 2.9 |
| Inventive Example 4C | 14.3 | 15.8 | 6.7 | 9.2 | 7.1 | 6.8 | 2.7 | 6.2 |
| Control Example 5A | 6.5 | 7.1 | n/a | n/a | 4.3 | 4.9 | 1.1 | 2.0 |
| Control Example 5B | 7.2 | 8.2 | n/a | n/a | 4.6 | 5.3 | 0.5 | 1.0 |

From the results above, it can be seen that the catalyst systems, and particularly those in Table 2 corresponding to the inventive examples and having a dehydrogenation catalyst comprising a mesoporous stannosilicate, provided a high selectivity to total olefins, including ethylene, propylene, and butenes. These selectivities, in view of the substantial per-pass conversion of propane, corresponded to economical and therefore commercially attractive product yields. Advantageously, selectivities to total paraffins, as well as selectivities to each of methane and ethane individually, for the inventive examples, were reduced significantly relative to the comparative examples. It was further observed that the Inventive Examples 4A-4C differed from the Comparative Example 3C with respect to the use of tin impregnated silica (optionally with additional promoter metal) as the dehydrogenation catalyst, as opposed to platinum impregnated silica, with tin promoter. The metathesis catalyst, and ratios of dehydrogenation catalyst to metathesis catalyst in the physical mixtures, were otherwise the same. Inventive Examples 4A-4C, however, provided up to a 2-fold improvement with respect to propane conversion over Comparative Example 3C, in addition to an increase in selectivity to desired C₂-C₄ olefins, but not C₅⁺ hydrocarbons, together with a significant decrease in selectivity to undesired methane. Moreover, these important performance advantages obtained in Inventive Examples 4A-4C relative to Comparative Example 3C, in terms of propane conversion and product selectivities, were maintained over the 8-hour test period, indicating that the inventive catalyst systems were stable did not significantly deactivate.

In addition to such favorable characteristics, catalyst systems described herein and comprising a dehydrogenation catalyst having a dehydrogenation active metal incorporated into a base silicate or other metal oxide framework can be advantageously regenerated without any appreciable performance deficit. This was demonstrated from the second and third cycle testing experiments, performed following first and second catalyst regenerations. The experiments allowed for a comparison of results obtained with dehydrogenation catalysts having varying amounts of tin, including those catalysts in the catalyst systems having bed configurations corresponding to Inventive Examples 3A-3D in which the amount of tin varied from 0.5 wt-% to 10 wt-%. Based on these results, stannosilicates with greater than 1 wt-% of tin incorporated into the silicate framework, such as greater than 3 wt-%, exhibited favorable performance parameters over stannosilicates with lower tin levels. These parameters included higher selectivities to, and yields of, the desired C₂-C₄ olefins, in addition to a lower selectivity to, and yield of, byproduct methane.

### Testing of Feeds Comprising H₂S

Experiments were performed to evaluate the effect of adding, or co-feeding, H₂S. The same catalyst pretreating and process conditions were used as described above for the testing of the catalyst systems provided in Table 2, with the exception of the WHSV (based on the total weight of catalyst in the catalyst system). For this process condition, values of 0.071 hr⁻¹, 0.094 hr⁻¹, and 0.141 hr⁻¹ were used in baseline examples, as well as examples in which the feed, in addition to propane, contained 25 or 50 vol-ppm H₂S. The baseline and sulfur addition examples are summarized below in Table 5.

**Table 5-Baseline and Sulfur Addition Examples**

| **Example** | **WHSV, Hr⁻¹** | **H₂S, Vol-ppm** |
|---|---|---|
| Baseline, Example 6A | 0.071 | 0 |
| Sulfur Addition, Example 6B | 0.071 | 25 |
| Baseline, Example 7A | 0.094 | 0 |
| Sulfur Addition, Example 7B | 0.094 | 25 |
| Baseline, Example 8A | 0.141 | 0 |
| Sulfur Addition, Example 8B | 0.141 | 25 |
| Baseline, Example 9A | 0.071 | 0 |
| Sulfur Addition, Example 9B | 0.071 | 50 |

For each of the examples summarized in Table 5 above, the catalyst system used included a bed of only a dehydrogenation catalyst, upstream of a bed of a physical mixture of dehydrogenation catalyst and metathesis catalyst, and a bed of only a metathesis catalyst, downstream of the bed of the physical mixture. These catalysts were in the form of extrudates (*i.e.,* cylindrical in form). With reference to the catalysts as described above in Table 1, this catalyst system is summarized in Table 6 below, including the weight ratio of catalysts used in the physical mixture, which is given in relative weights of [dehydrogenation catalyst] : [metathesis catalyst].

**Table 6-Catalyst System Used in the Baseline and Sulfur Addition Examples**

| **BED-DH Dehydrogenation Catalyst** | **BED-PM Physical Mixture** | **BED-M Metathesis Catalyst** | **Bed Weight Ratios [DH]:[PM]:[M]** |
|---|---|---|---|
| DEHY-INV D | DEHY-INV D / META C | META C | [1]:[1]:[1] |
| | at 1:1 w/w | | |

For each of the baseline and sulfur addition examples, this catalyst system was evaluated as described above with respect to testing of the catalyst systems described in Table 2. Conversion and selectivities to olefin products were determined from effluent samples obtained 3 hours after introduction of the propane feed (*i.e.,* at 3 hours on stream), either lacking the H₂S co-feed ("Baseline") or containing this co-feed ("Sulfur Add."). These performance parameters are provided below in Table 7.

**Table 7-Conversion / Olefin Product Selectivities, Baseline and Sulfur Addition Examples**

| **Example** | **% Propane Conversion** | **% Selectivity, Total Olefins** | **% Selectivity, Propylene** | **% Selectivity, Ethylene** | **% Selectivity, Butenes** |
|---|---|---|---|---|---|
| Baseline, Example 6A | 43.7 | 88.9 | 41.8 | 20.3 | 16.8 |
| Sulfur Add., Example 6B | 43.9 | 89.8 | 46.4 | 17.5 | 17.4 |
| Baseline, Example 7A | 37.5 | 90.3 | 42.4 | 20.8 | 17.2 |
| Sulfur Add., Example 7B | 37.0 | 90.6 | 43.5 | 20.0 | 18.1 |
| Baseline, Example 8A | 29.1 | 92.7 | 44.3 | 20.7 | 18.5 |
| Sulfur Add., Example 8B | 29.6 | 93.8 | 46.3 | 19.6 | 19.4 |
| Baseline, Example 9A | 36.1 | 92.4 | 43.4 | 21.9 | 17.1 |
| Sulfur Add., Example 9B | 37.4 | 93.1 | 46.8 | 19.6 | 18.5 |

Also determined from the sample analyses were the selectivities to undesired, low value byproducts, including paraffins. The selectivity to all paraffins, as well as the selectivities to the individual paraffins, methane and ethane, which result from cracking and/or hydrogenation side reactions, are provided below in Table 8. This table also provides the selectivities to hydrocarbon byproducts having 5 or more carbon atoms, *i.e.,* C₅⁺ hydrocarbon byproducts ("C₅⁺ HCBNS").

**Table 8-Paraffin / C₅⁺ Hydrocarbon Selectivities, Baseline and Sulfur Addition Examples**

| **Example** | **% Selectivity Total Paraffins** | **% Selectivity, Ethane** | **% Selectivity, Methane** | **% Selectivity**, **C₅⁺ HCBNS** |
|---|---|---|---|---|
| Baseline, Example 6A | 11.1 | 5.4 | 4.9 | 10.1 |
| Sulfur Add., Example 6B | 10.2 | 4.7 | 4.9 | 8.5 |
| Baseline, Example 7A | 9.7 | 4.3 | 4.8 | 9.8 |
| Sulfur Add., Example 7B | 9.4 | 4.1 | 4.8 | 9.0 |
| Baseline, Example 8A | 7.3 | 2.8 | 3.9 | 9.2 |
| Sulfur Add., Example 8B | 6.2 | 2.5 | 3.8 | 7.8 |
| Baseline, Example 9A | 7.6 | 2.9 | 4.3 | 9.9 |
| Sulfur Add., Example 9B | 6.9 | 2.5 | 4.0 | 8.0 |

It is evident from the results above that the H₂S did not cause deactivation (poisoning) of the catalyst system, despite this contaminant being problematic in the case of conventional dehydrogenation catalysts containing noble metals such as Pt. Moreover, the addition of this sulfur-bearing compound actually had a beneficial effect in terms of increasing the selectivities to, and yields of, desired olefin products (C₂-C₄ olefins), as well as decreasing the selectivities to, and yields of, paraffin byproducts and particularly methane and ethane. These selectivity and yield advantages were achieved despite any appreciable differences in propane conversion. Directionally, the higher H₂S concentration of 50 vol-ppm resulted in an increase in selectivity to C₂-C₄ olefins, compared to that obtained at the lower H₂S concentration of 25 vol-ppm. The C₂-C₄ olefin yield at the higher H₂S concentration was 6.9% for these experiments, compared to only 3.6% obtained at the lower H₂S concentration. The selectivity to these olefins was likewise increased at the higher H₂S concentration. The improvements observed with the addition of H₂S are believed to result from a suppression, by the sulfur-bearing compound, of undesired side reactions (*e.g*., cracking and hydrogenation). Such side reactions lead to decreased olefin production and increased paraffin byproduct production.

### Testing of Liquefied Petroleum Gas (LPG) Feed

Experiments were performed to evaluate the conversion of a feed characteristic of commercial LPG obtained from a refinery and that contained propane, n-butane, and i-butane, and n-butane at volume percentages of 54.6%, 23.1%, and 22.2%, respectively, in addition to trace amounts of mixed butenes and pentenes. The same catalyst pretreating and process conditions were used as described above for the testing of the catalyst systems provided in Table 2. The catalyst system used for the conversion of LPG to olefins was the 3-bed system described in Table 6 and used for the testing of feeds comprising H₂S. In the evaluation of this catalyst system, samples of the effluent exiting the downstream bed of the catalyst designated META C (Table 1) were obtained after 3 hours on stream and 10 hours on stream. The samples were quantitatively analyzed for their composition in order to determine propane conversion and selectivity to all olefins (including olefins having 5 carbon atoms or greater, C₅⁺), as well as the selectivities to the individual olefins, propylene and ethylene, and selectivity to butene isomers (C₄ olefins). The sample analyses were performed as described above for the testing of the catalyst systems provided in Table 2. Yields of all olefins, as well as yields of propylene, ethylene, and butene isomers can be determined as described above, using the analytical results that allow for determination of propane conversion and respective selectivities to all olefins and individual olefin species.

The following conversion and selectivities to olefin products, as determined from the experimental results, are provided below in Table 9.

**Table 9 Conversion and Selectivities to Olefin Products, LPG Feed**

| **Example** | **% Propane Conversion** | | **% Selectivity, Total Olefins** | | **% Selectivity, Propylene** | | **% Selectivity, Ethylene** | | **% Selectivity, Butenes** | |
|---|---|---|---|---|---|---|---|---|---|---|
| | 3hr | 10hr | 3hr | 10hr | 3hr | 10hr | 3hr | 10hr | 3hr | 10hr |
| LPG Feed Example 10 | 38.2 | 38.0 | 92.2 | 91.1 | 31.2 | 31.4 | 9.8 | 11.0 | 39.1 | 34.6 |

Also determined from the sample analyses, but not shown in Table 9, were the selectivities to undesired, low value byproducts, including paraffins. The selectivity to all paraffins, as well as the selectivities to the individual paraffins, methane and ethane, which result from cracking and/or hydrogenation side reactions, are provided below in Table 10. Also provided in this Table are the selectivities to hydrocarbon byproducts having 5 or more carbon atoms, *i.e.,* C₅⁺ hydrocarbon byproducts ("C₅⁺ HCBNS").

**Table 10 Paraffin / C₅⁺ Hydrocarbon Selectivities, LPG Feed**

| **Example** | **% Selectivity Total Paraffins** | | **% Selectivity, Ethane** | | **% Selectivity, Methane** | | **% Selectivity, C₅⁺ HCBNS** | |
|---|---|---|---|---|---|---|---|---|
| | 3hr | 10hr | 3hr | 10hr | 3hr | 10hr | 3hr | 10hr |
| LPG Feed Example 10 | 7.8 | 8.9 | 3.6 | 4.2 | 4.2 | 4.7 | 12.1 | 14.1 |

These results demonstrated the effectiveness of catalyst systems described herein for the conversion of readily available mixtures of paraffinic hydrocarbons, such as LPG, to valuable olefins with favorable selectivities and yields. Advantageously, selectivities to total paraffins were maintained below 10%, whereas selectivities to each of methane and ethane were maintained below 5%.

### Preparation and Testing of Dehydrogenation and Metathesis Catalyst Particles

A dehydrogenation and metathesis catalyst, having tungsten as an olefin metathesis active metal deposited onto a mesoporous stannosilicate support, was prepared in a manner similar to that described above with respect to the preparation of the catalyst designated DEHY-INV C, but with the additional introduction of a source of the tungsten. In particular, cetyltrimethylammonium bromide (CTAB) was dissolved in an ethanol-water solvent by combining these components and stirring until the mixture became a homogeneous solution. Aqueous ammonium hydroxide was then added to this solution to adjust its pH value to within 8-12, followed by a slow addition of tetraethylorthosilicate (TEOS) and SnCl₂·2H₂O. The amount of Sn, ultimately deposited onto the base material, or base silicate framework, of the dehydrogenation and metathesis catalyst, was controlled to achieve a target of 5 wt-%, by adjusting the amounts of TEOS and SnCl₂·2H₂O. Following the complete addition of the silica and tin sources, the reaction was continued for a period of 3-24 hours. Thereafter, a controlled amount of ammonium metatungstate monohydrate, as necessary to provide 8 wt-% WO₂ on the catalyst, was added and the reaction was continued for 2-12 hours. The resulting, precipitated solids were filtered, washed several times with deionized water, and dried at room temperature initially and at 120°C (248°F) for 12 hours subsequently. Finally, the dehydrogenation and metathesis catalyst was obtained by calcining at 550°C (1022°F) for 6 hours. For purposes of testing in the experiments described below, this catalyst, having both dehydrogenation and metathesis active metals present in a solid support and being prepared by a precipitation method as described herein, is referred to in Tables 11 and 12 as "InvEx11A, Precip-Single."

Another dehydrogenation and metathesis catalyst was prepared by impregnation of an SiO₂ support using an incipient wetness impregnation method and impregnation solutions of tin (II) chloride dehydrate and ammonium metatungstate monohydrate to disperse both Sn and W metals onto the support. Following immersion in the impregnation solutions, the impregnated support was then dried at 120°C (248°F) for 12 hours and calcined under air at 550°C (1022°F) for 8 hours to obtain the catalyst, which contained, based on the total catalyst weight, 5 wt-% Sn and 8 wt-% W, with the balance being SiO₂. For purposes of testing in the experiments described below, this catalyst, having both dehydrogenation and metathesis active metals present in a solid support and being prepared by an impregnation method as described herein, is referred to in Tables 11 and 12 as "InvEx11B, Impreg-Single."

To provide a paraffin dehydrogenation and metathesis catalyst system, separate types of dehydrogenation catalyst and metathesis catalyst, and in particular the catalysts designated in Table 1 above as DEHY-INV C and META A, were physically mixed. In particular, this system was formed using 1 gram each of the separate catalyst types. For purposes of testing in the experiments described below, this catalyst system, being a physical mixture of both a dehydrogenation catalyst and a metathesis catalyst as described herein, is referred to in Tables 11 and 12 as "InvEx11C, Separate."

For comparative purposes, a catalyst was prepared by impregnation of an SiO₂ support using an incipient wetness impregnation method and impregnation solutions of chloroplatinic acid, tin (II) chloride dehydrate, and ammonium metatungstate monohydrate to disperse Pt, Sn, and W metals onto the support. Following immersion in the impregnation solutions, the impregnated support was then dried at 120°C (248°F) for 12 hours and calcined under air at 550°C (1022°F) for 8 hours to obtain the catalyst, which contained, based on the total catalyst weight, 0.6 wt-% Pt, 0.6 wt-% Sn, and 8 wt-% W, with the balance being SiO₂. The platinum loading was purposefully twice that of the catalyst designated in Table 1 above as DEHY-COMP C, having 0.3 wt-% Pt, to confirm that increasing Pt would not appreciably impact performance. For purposes of testing in the experiments described below, this catalyst, having both Pt as a conventional dehydrogenation active metal, in addition to Sn and W was dehydrogenation active and metathesis active metals, respectively, present in a solid support and being prepared by an impregnation method as described herein, is referred to in Tables 11 and 12 as "CompEx11D, Impreg-Single."

Experiments were carried out, in Examples 11A, 11B, and 11D, to evaluate the performance of the single types of dehydrogenation and metathesis catalysts, namely InvEx11A, Precip-Single; InvEx11B, Impreg-Single; and CompEx11D, Impreg-Single, as described above. In addition, an experiment was carried out, in Example 11C, to evaluate the performance of the physical mixture of separate types of dehydrogenation catalyst and metathesis catalyst, namely InvEx11C, Separate. Whereas the physical mixture was formed of 1 gram of each of the catalysts designated in Table 1 above as DEHY-INV C and META A, the catalyst beds of the single types of dehydrogenation and metathesis catalyst particles each contained 2 grams of these particles. The same catalyst pretreating and process conditions were used as described with respect to the testing of the catalyst systems provided in Table 2, with the exception of the WHSV, which was 0.845 hr⁻¹ for Inventive Examples 11A-11C and Comparative Example 11D, based on the total 2 grams of catalyst used in each case. In the performance evaluation, propane was used as the feed, and samples of the effluent exiting the respective beds of (i) the single type of dehydrogenation and metathesis catalyst (Inventive Examples 11A and 11B, and Comparative Example 11D) and (ii) the physical mixture of separate types of dehydrogenation catalyst and metathesis catalyst (Inventive Example 11C), were obtained after 1 hour on stream and 8 hours on stream. The samples were quantitatively analyzed for their composition in order to determine propane conversion and selectivity to all olefins (including olefins having 5 carbon atoms or greater, C₅⁺), as well as the selectivities to the individual compounds, propylene and ethylene, and selectivity to butene isomers (C₄ olefins). The sample analyses were performed as described above with respect to the testing of the catalyst systems provided in Table 2. Yields of all olefins, as well as yields of propylene, ethylene, and butene isomers can be determined as described above, using the analytical results that allow for determination of propane conversion and respective selectivities to all olefins and individual olefin species.

The following conversion and selectivities to olefin products, as determined from the experimental results, are provided below in Table 11.

**Table 11-Conversion / Olefin Product Selectivities, Single and Separate Catalyst Types**

| **Example** | **% Propane Conversion** | | **% Selectivity, Total Olefins** | | **% Selectivity, Propylene** | | **% Selectivity, Ethylene** | | **% Selectivity, Butenes** | |
|---|---|---|---|---|---|---|---|---|---|---|
| Catalyst Type | 1hr | 8hr | 1hr | 8hr | 1hr | 8hr | 1hr | 8hr | 1hr | 8hr |
| InvEx11A Precip-Single | 23.5 | 24.9 | 88.4 | 88.0 | 71.5 | 70.2 | 11.2 | 11.0 | 4.2 | 5.2 |
| InvEx11B Impreg-Single | 25.4 | 27.8 | 89.7 | 88.2 | 82.2 | 79.7 | 5.6 | 5.9 | 0.6 | 1.0 |
| InvEx11C Separate | 26.1 | 27.0 | 88.7 | 88.2 | 77.9 | 75.9 | 8.9 | 9.0 | 1.1 | 2.4 |
| CompEx11D Impreg-Single | 14.5 | 12.6 | 85.5 | 82.1 | 58.5 | 43.7 | 16.8 | 25.2 | 4.7 | 5.7 |

Also determined from the sample analyses, but not shown in Table 11, were the selectivities to undesired, low value byproducts, including paraffins. The selectivity to all paraffins, as well as the selectivities to the individual paraffins, methane and ethane, which result from cracking and/or hydrogenation side reactions, are provided below in Table 12. Also provided in this Table are the selectivities to hydrocarbon byproducts having 5 or more carbon atoms, *i.e.,* C₅⁺ hydrocarbon byproducts ("C₅⁺ or HCBNS").

**Table 12-Paraffin / C₅⁺ Hydrocarbon Selectivities, LPG Feed**

| **Example** | **% Selectivity Total Paraffins** | | **% Selectivity, Ethane** | | **% Selectivity, Methane** | | **% Selectivity, C₅⁺ HCBNS** | |
|---|---|---|---|---|---|---|---|---|
| Catalyst Type | 1hr | 8hr | 1hr | 8hr | 1hr | 8hr | 1hr | 8hr |
| InvEx11A Precip-Single | 11.6 | 12 | 4.4 | 4.9 | 6.9 | 6.8 | 1.5 | 1.8 |
| InvEx11B Impreg-Single | 10.3 | 11.8 | 4.8 | 5.6 | 5.3 | 5.6 | 1.3 | 1.7 |
| Catalyst Type | 1hr | 8hr | 1hr | 8hr | 1hr | 8hr | 1hr | 8hr |
| InvEx11C Separate | 12.1 | 11.8 | 4.4 | 4.9 | 6.8 | 6.7 | 0.7 | 1.0 |
| CompEx11D Impreg-Single | 14.5 | 17.9 | 4.6 | 4.6 | 9.4 | 12.8 | 5.4 | 6.5 |

From the results above, it can be appreciated that advantages may be obtained using catalysts in which both the dehydrogenation active metal(s) and olefin metathesis active metal(s) are present together on a single type of solid support, whether prepared by precipitation or impregnation methods as described herein. These are namely the same advantages, in terms of favorable propane conversion and selectivities to desired olefins, with relatively low selectivities to paraffins and other unwanted byproducts, as obtained using a physical mixture of separate catalyst types for performing dehydrogenation and metathesis. Among these Examples 11A-11D, it was apparent that the single catalyst having platinum as a dehydrogenation active metal, *i.e*., CompEx11D, Impreg-Single, exhibited the poorest performance in terms of propane conversion, selectivity to total olefins, and stability (based on the conversion loss over 8 hours of testing). The inferior results in Example 11D were obtained despite the elevated platinum loading of 0.6 wt-%, compared to amounts conventionally used. Whereas use of the single dehydrogenation and metathesis catalyst tested in Example 11A, *i.e*., InvEx11A, Precip-Single, resulted in a lower total olefin yield compared to use of the catalyst system formed as a physical mixture in Example 11 C, *i.e*., InvEx11C, Separate, use of the single catalyst nonetheless resulted in higher selectivities to both ethylene and butenes.

### Long-Term Stability Testing

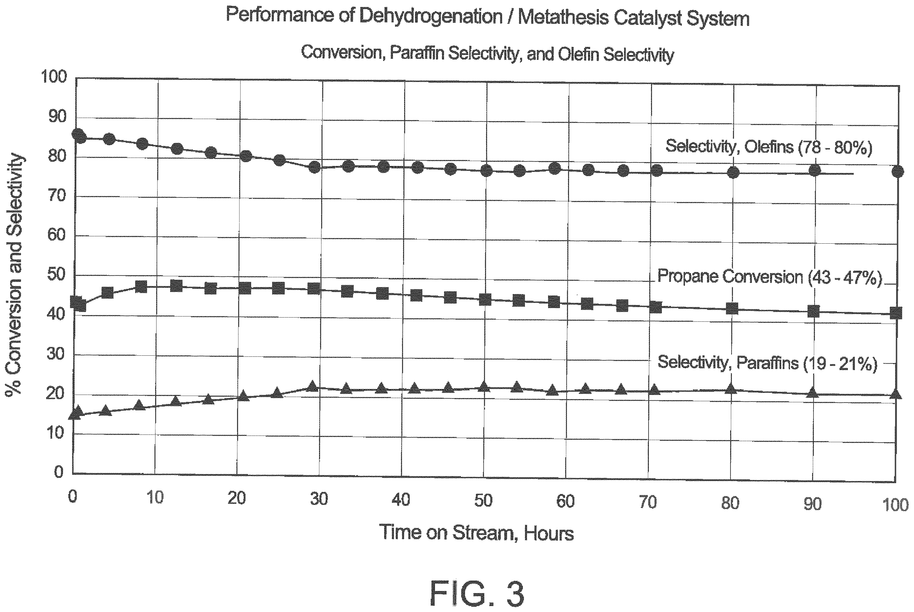

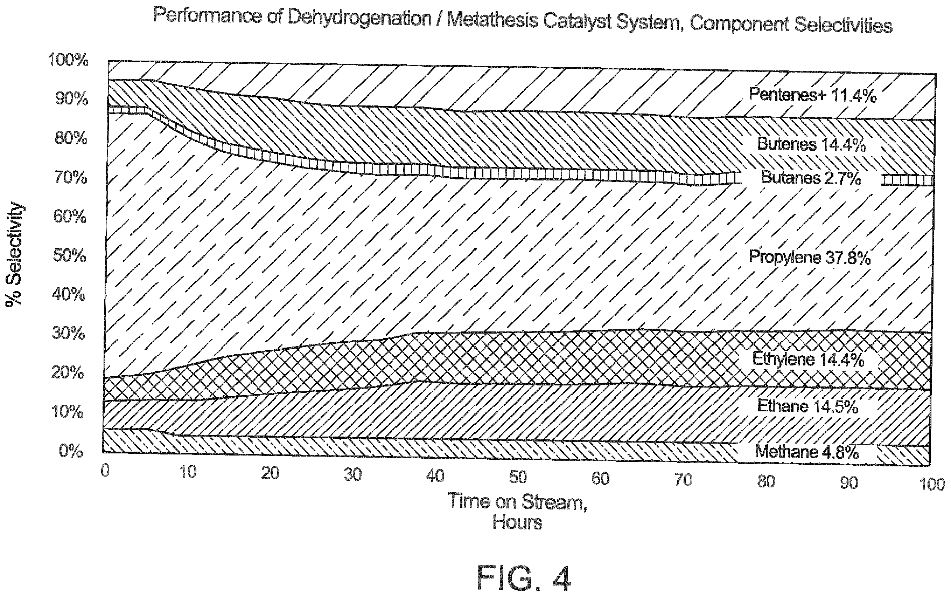

Experiments were performed to evaluate the long-term stability for propane dehydrogenation and metathesis, of a catalyst system that included (i) a bed of 20 grams of only the mesoporous stannosilicate dehydrogenation catalyst designated DEHY-INV D in Table 1, upstream of (ii) a bed of a physical mixture of (A) 10 grams of the mesoporous stannosilicate dehydrogenation catalyst designated DEHY-INV D in this table and (B) 10 grams of the metathesis catalyst designated META C in this table, and (iii) a bed of 20 grams of only the metathesis catalyst designated META C, downstream of the bed of the physical mixture. This catalyst system was loaded into a tubular fixed-bed stainless steel reactor with an inner diameter of 25 mm (1 inch). The same catalyst pretreating with O₂ and then H₂, was used as described above for the testing of the catalyst systems described in Table 2. Following the pretreating, a flow of 40-160 ml/min of propane feed was contacted the catalyst system, with the bed temperature maintained at 570-600°C (1058-1112°F). The purity of the propane feed was 93-99%, and conversion and selectivities to olefin products were determined from effluent samples obtained from the catalyst system, following analysis by GC-FID as described above. Performance was evaluated over an extended testing period of 100+ hours. FIG. 3 depicts the propane conversion, selectivity to total olefins, and selectivity to paraffins over this operating period. FIG. 4 depicts the selectivities over time to the component olefins (ethylene, propylene, butenes, and pentenes) and component paraffins (methane, ethane, and butanes). These experiments demonstrated that the catalyst system achieved an exceptionally stable transformation of propane, over an extended operation period, to a variety of olefins having differing carbon numbers and especially the valuable C₂-C₄ olefins. In similar, long-term performance testing, it was demonstrated that the catalyst system could be utilized to attain, over a period exceeding 100 hours of operation, at least 30% conversion of propane and greater than 75% selectivity to all olefins (with at least 65% selectivity to C₂-C₄ olefins and less than 15% selectivity to C₅ olefins) at 590°C (1094°F), 1 bar absolute pressure, and 0.07-0.30 hr⁻¹ WHSV based on the total weight of the catalyst in the system and 0.14-0.60 hr⁻¹ WHSV based on the weight of the dehydrogenation catalyst. In contrast, the mesoporous stannosilicate dehydrogenation catalyst, when tested in isolation, converted the feed primarily to propylene with a selectivity of >85%.

### H₂-TPR for Characterization of Sn Dispersion

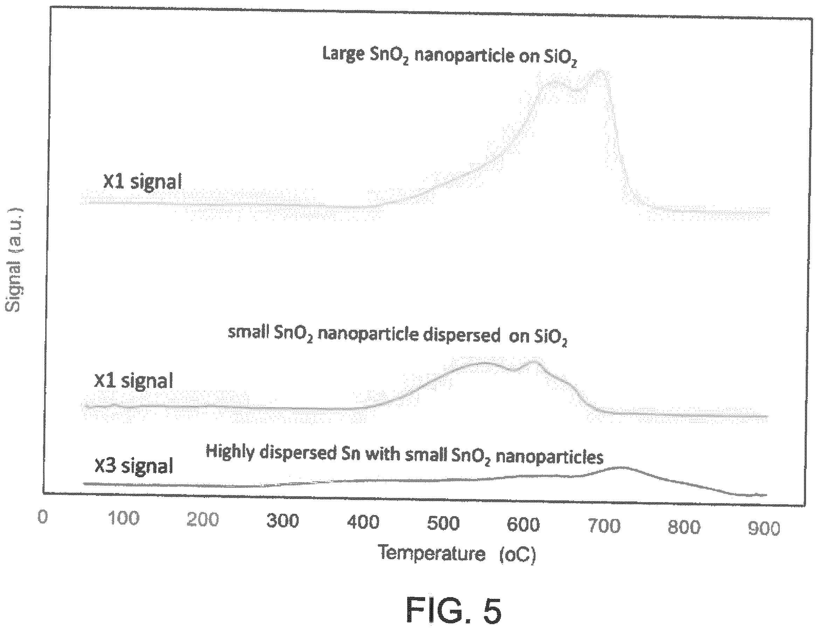

Hydrogen-temperature programmed reduction (H₂-TPR) can be used to characterize the dispersion of Sn on catalyst base materials, such as those comprising silica (SiO₂) or silicate anions. A standard method for performing this analysis is described above. The reduction peaks of SnO₂ can be divided into two ranges, with the peak in the range of 200-450°C corresponding to reduction of Sn⁴⁺, for example in nanoparticles or surface species, to Sn²⁺, and the peak in the range of 600-800°C corresponding to reduction of Sn⁴⁺ or Sn²⁺ to metallic tin, Sn⁰. FIG. 5 shows the differences in H₂-TPR profiles, obtained for Sn/SiO₂ catalyst samples with the same tin loading, but prepared by different precipitation and impregnation methods. In the case of the lower profile ("x3 signal," referring to a factor of 3 amplification), the Sn is highly dispersed due to bond formation with the base material (*e.g.,* by the formation of -Si-O-Sn- bonds or -O-Si-O(Sn-O-Sn)ₙO-Si- bonds), as exhibited by the small areas under the curve (reduction peaks) in both temperature ranges. Only a relatively small contribution of the loaded tin is in the form of SnO₂ nanoparticles that are reduced by this method. In the middle profile (x1 signal-small SnO₂), it can be seen that a larger contribution of the loaded tin exists in the form of SnO₂ nanoparticles that are reducible above 450°C. Both the lower and middle profiles were obtained with samples of stannosilicates prepared by coprecipitation as described herein. In contrast, the upper profile (xl signal-large SnO₂) was obtained with a sample of a stannosilicate prepared by incipient wetness impregnation and having Sn substantially in the form of larger SnO₂ nanoparticles that were mostly reducible to metallic tin in the 600-800°C temperature range. Therefore, the H₂-TPR analysis demonstrated how the catalyst preparation method could be used to influence the types of Sn species obtained, namely Sn-O-Si species incorporated into the base material versus SnO₂ nanoparticles external to pores of the base material, and their relative amounts.

Overall aspects of the invention are directed to processes that utilize the catalysts and catalyst systems as described herein to convert paraffins to a variety of desired, higher value olefins. These catalysts and catalyst systems lead to various improvements, such as increased selectivities to, and yields of, desired olefin products (*e.g.,* C₂-C₄ olefins), decreased selectivities to, and yields of, undesired byproducts (*e.g*., methane and/or ethane), with high stability, including stability over multiple catalyst regenerations. Feeds additionally comprising a sulfur-bearing compound (*e.g.,* H₂S generated *in situ* or already present as a feed contaminant) can provide additional advantages as described herein. Those having skill in the art, with the knowledge gained from the present disclosure, will recognize that various changes can be made in the above catalysts, catalyst systems, and processes using these catalyst systems, without departing from the scope of the present disclosure.

## Claims

1. A paraffin dehydrogenation and metathesis catalyst system comprising:
a dehydrogenation catalyst comprising a stannosilicate, and
a metathesis catalyst comprising one or more olefin metathesis active metals selected from the group consisting of metals in Group 6 and Group 7 of the periodic table, wherein said one or more olefin metathesis active metals are supported on an olefin metathesis solid support.

2. The system of claim 1, wherein the stannosilicate comprises tin being incorporated into a silicate or aluminosilicate framework.

3. The system of claim 1 or 2, wherein tin is present in the dehydrogenation catalyst in an amount from about 3 wt-% to about 15 wt-%.

4. The system of claim 3, wherein the dehydrogenation catalyst further comprises one or more additional metals selected from the group consisting of In, Pb, Ge, Ga, Tl, Cu, Ag, Au, and mixtures thereof, being present in an amount, or combined amount, from about 0.1 wt-% to about 6 wt-%.

5. The system of any of the preceding claims, wherein the one or more olefin metathesis active metals are selected from the group consisting of W, Mo, and Re.

6. The system of any one of the preceding claims, wherein the dehydrogenation catalyst and the metathesis catalyst are physically mixed in a catalyst bed.

7. The system of claim 6, further comprising a second catalyst bed comprising substantially all of the metathesis catalyst.

8. The system of any one of the claims 1 to 5, wherein the dehydrogenation catalyst and the metathesis catalyst are in separate catalyst beds.

9. The system of claim 8, comprising three or more of said separate catalyst beds, alternatingly comprising said dehydrogenation catalyst and the metathesis catalyst.

10. A dehydrogenation and metathesis process, the process comprising contacting a feed comprising a paraffinic hydrocarbon with at least one catalyst bed comprising a physical mixture of:
a dehydrogenation catalyst comprising one or more dehydrogenation active metals selected from the group consisting of metals in Group 11, Group 13, and Group 14 of the periodic table, wherein said one or more dehydrogenation active metals are supported on a dehydrogenation solid support, and
a metathesis catalyst comprising one or more olefin metathesis active metals selected from the group consisting of metals in Group 6 and Group 7 of the periodic table, wherein said one or more olefin metathesis active metals are supported on an olefin metathesis solid support.

11. The process of claim 10, wherein a combined amount of metals in Groups 8-10 of the periodic table is less than about 0.1 wt-% in the dehydrogenation catalyst.

12. The process of claim 10 or claim 11, wherein the dehydrogenation solid support comprises a stannosilicate, having tin incorporated into a silicate framework.

13. The process of claim 10 or claim 11, wherein the dehydrogenation solid support comprises a dehydrogenation support metal oxide, a dehydrogenation support zeolite, or a combination thereof.

14. The process of claim 13, wherein the dehydrogenation support metal oxide is selected from the group consisting of alumina, silica, zirconia, titania, magnesia, calcium oxide, and mixtures thereof.

15. The process of claim 14, wherein the dehydrogenation support metal oxide is an oxide of a first metal selected from the group consisting of Li, Mg, Zn, Fe, Ca, Ni, Co, Mn, and Cu, and wherein the dehydrogenation solid support comprises a further dehydrogenation support metal oxide of a second metal selected from the group consisting of Al, Ga, Y, In, Fe, Co, Ni, Mn, Cr, Ti, V, Zr, and La.
